(19) Europäisches Patentamt
European Patent Office
Office européen des brevets

(11) Veröffentlichungsnummer: **0 051 819**
**B1**

(12) # EUROPÄISCHE PATENTSCHRIFT

(45) Veröffentlichungstag der Patentschrift: **13.02.85**

(21) Anmeldenummer: **81109200.6**

(22) Anmeldetag: **29.10.81**

(51) Int. Cl.⁴: **C 07 C 49/84**, A 61 K 31/12, A 61 K 31/165, A 61 K 31/66, C 07 C 103/76, C 07 C 103/78, C 07 C 103/82, C 07 C 131/00, C 07 C 143/36, C 07 C 151/00, C 07 C 153/063

(54) **Tetra-substituierte Benzolderivate, deren Herstellung und pharmazeutische Präparate damit.**

(30) Priorität: **12.11.80 GB 8036223**
**05.10.81 GB 8130001**

(43) Veröffentlichungstag der Anmeldung: **19.05.82 Patentblatt 82/20**

(45) Bekanntmachung des Hinweises auf die Patenterteilung: **13.02.85 Patentblatt 85/7**

(84) Benannte Vertragsstaaten: **AT BE CH DE FR GB IT LI LU NL SE**

(56) Entgegenhaltungen:
**EP - A - 0 013 960**
**DD - A - 146 295**
**US - A - 3 087 821**
**US - A - 4 032 635**
**US - A - 4 219 569**

**Chemical Abstracts Band 94, Nr. 13, 30. März 1981, Columbus, Ohio, USA J.K. MAKRANDI et al. "A new biogenetic approach for the synthesis of homoiso-flavanones", Seite 720, Abstract Nr. 103122m**
**JOURNAL OF THE CHEMICAL SOCIETY, Nr. 10, 1980, London B.C.B. BEZUIDENHOUDT et al. "Novel alpha-Methyldeoxybenzoins from the Heartwood of Pterocarpus angolensis D.C.: Absolute Configuration**

(73) Patentinhaber: **F. HOFFMANN-LA ROCHE & CO. Aktiengesellschaft, CH-4002 Basel (CH)**

(72) Erfinder: **Shinma, Nobuo, 403 Nagase Building, Kamigo-cho 100, Totsuka-ku, Yokohama-shi, Kanagawa-ken (JP)**
Erfinder: **Fujiu, Morio, 5-7-203, Zengyo Danchi, 3768-3, Fujisawa, Fujisawa-shi, Kanagawa-ken (JP)**
Erfinder: **Umeda, Isao, 204 Heights Hakuraku, 4-4-20, Rokkakubashi, Yokohama-shi, Kanagawa-ken (JP)**
Erfinder: **Ohtsuka, Tatsuo, 615 Kamimachiya, Kamakura-shi, Kanagawa-ken (JP)**
Erfinder: **Ishitsuka, Hideo, 1-1-405, Katsura-cho, 1-banchi, Totsuka-ku, Yokohama-shi, Kanagawa-ken (JP)**
Erfinder: **Suhara, Yasuji 9-506, Dream Height, 1403-banchi, Matano-cho Totsuka-ku, Yokohama-shi, Kanagawa-ken (JP)**

(74) Vertreter: **Lederer, Franz, Dr. et al, Patentanwälte Dr. Franz Lederer Reiner F. Meyer Lucile-Grahn-Strasse 22, D-8000 München 80 (DE)**

(56) Entgegenhaltungen:
**and Conformation of the first Sesquiterpenylangolensis, and X-Ray Crystal Structure of 4-0-alpha-Cadinylangolensin", Seiten 2179 bis 2183**
**JOURNAL OF ORGANIC CHEMISTRY Band 41, Nr. 10, 14. Mai 1976, London J.R. COLE et al. "Uvaretin, a New Antitumor Agent from Uvaria acuminata (Annonaceae)", Seiten 1852 bis 1855**
**JOURNAL OF ORGANIC CHEMISTRY Band 42, Nr. 8, 15. April 1977, London W.L. LASSWELL JR. et al. "Cytotoxic C-Benzylated Flavonoids from Uvaria chamae", Seiten 1295 bis 1302**
**Chemical Abstracts Band 92, Nr. 10 10. März 1980, Columbus, Ohio, USA S.D. MANCINI et al. "Cytotoxic principles from the sap of Kalmia latifolia", Seiten 379, Abstract Nr. 82292h**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die vorliegende Erfindung betrifft neue tetra-substituierte Benzolderivate, ein Verfahren zu ihrer Herstellung und antivirale Mittel mit diesen Verbindungen.

Die Erfindung betrifft insbesondere neue tetra-substituierte Benzolderivate der Formel

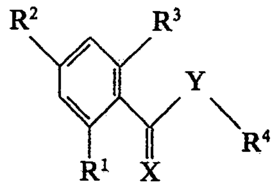

(I)

worin X Sauerstoff, Schwefel oder Hydroxyimino; Y Methylen oder Imino; $R^1$ Hydroxy, Phosphonooxy, Glycosyloxy, acyliertes Glycosyloxy, Acyloxy oder nieder-Alkoxycarbonyloxy; $R^2$ nieder-Alkoxy, nieder-Alkenyloxy oder nieder-Alkylthio; $R^3$ nieder-Alkoxy; $R^4$ nieder-Alkyl, p-nieder-Alkoxybenzoyl oder substituiertes oder unsubstituiertes Phenyl, Benzyl, Pyridylmethyl, Furfuryl, Tetrahydrofurfuryl, Thienyl, Tetrahydrothenyl, Pyrrolylmethyl, Pyrrolinylmethyl oder Pyrrolidinylmethyl darstellt, mit der Bedingung, daß Y nicht Methylen ist, wenn $R^4$ Alkyl, Phenyl, substituiertes Phenyl, Benzyl oder p-Hydroxybenzyl ist und mit der weiteren Bedingung, daß $R^2$ und $R^3$ voneinander verschieden sind, wenn $R^4$ p-Methoxybenzyl und Y Methylen ist,

und pharmazeutisch anwendbare Salze davon.

Im europäischen Patent Nr. 13 960 werden Benzolderivate mit antiviralen Eigenschaften beschrieben. Die erfindungsgemäßen Verbindungen unterscheiden sich von diesen bekannten Verbindungen dadurch, daß Y in der Bedeutung Methylen oder Imino vorliegt und somit eine Doppelbindung fehlt.

Bevorzugte Beispiele von Glycosyloxy und acylierten Glycosyloxyresten sind $\beta$-D-Glucopyranosyloxy und Tetra-O-acetyl-$\beta$-D-glucopyranosyloxy. Acyloxyreste leiten sich vorzugsweise von aliphatischen Säuren mit 2—18 C-Atomen oder von aromatischen Säuren ab, bevorzugte Acyloxyreste sind Acetoxy, Propionyloxy, Butyryloxy, Isobutyryloxy, Pivaloyloxy, Stearoyloxy und Benzoyloxy. Nieder-alkoxycarbonylreste enthalten vorzugsweise bis zu 7 C-Atome, ein bevorzugter Alkoxycarbonyloxyrest ist Ethoxycarbonyloxy. Nieder-alkoxyreste enthalten vorzugsweise 1—6 C-Atome, insbesondere 1—4 C-Atome wie Methoxy, Ethoxy, Propoxy, Isopropoxy und Butoxy. Nieder-alkylthioreste enthalten vorzugsweise 1—6 C-Atome, insbesondere 1—4 C-Atome, wie Methylthio. Nieder-alkylreste enthalten vorzugsweise 1—6 C-Atome, insbesondere 1—4 C-Atome, wie Methyl, Ethyl, Propyl und Butyl. Niederalkenyloxyreste enthalten vorzugsweise 2—7 C-Atome, insbesondere 2—4 C-Atome wie Allyloxy und 3-Methyl-2-propenyloxy. Bevorzugte substituierte Phenyl-, Benzyl- und Pyridylmethylreste sind nieder-Alkoxyphenyl wie p-Methoxyphenyl; Benzyl, das durch ein oder mehrere Substituenten wie Hydroxy, Halogen, nieder-Alkyl, nieder-Alkoxy, nieder-Alkylthio, Amino, Dialkylamino, Allyloxy, Benzyxloxy und Alkylendioxy substituiert ist, insbesondere p-Hydroxybenzyl, m-Hydroxy-p-methoxybenzyl, p-Chlorbenzyl, p-Methylbenzyl, p-Methoxybenzyl, m-Methoxybenzyl, m,p-Dimethoxybenzyl, p-Butoxybenzyl, p-(Methylthio)benzyl, p-(Dimethylamino)benzyl, p-(Allyloxy)-benzyl, p-(Benzyloxy)benzyl und m,p-(Methylendioxy)-benzyl. Ein Beispiel eines Pyridylmethylrestes ist 4-Pyridylmethyl. Bevorzugte substituierte Furfuryl, Thenyl und Pyrrolylmethylreste und gesättigte Derivate davon sind Furfuryl, 5-Methylfurfuryl, Tetrahydro-5-methylfurfuryl, 2-Thenyl, Tetrahydro-2-thenyl und 2-Pyrrolylmethyl.

Eine bevorzugte Gruppe von Verbindungen der Formel I sind die, in denen $R^2$ nieder-Alkoxy oder nieder-Alkylthio sind und $R^1$, $R^3$, $R^4$, X und Y die obige Bedeutung haben.

Weiterhin bevorzugte Verbindungen der Formel I sind die, in denen $R^1$ Hydroxy, nieder-Alkanoyloxy, Benzoyloxy, Phosphonooxy, nieder-Alkoxycarbonyloxy; $R^2$ nieder-Alkoxy oder nieder-Alkenyloxy; $R^3$ nieder-Alkoxy; $R^4$ substituiertes Phenyl; X Sauerstoff oder Schwefel und Y Methylen oder Imino ist, insbesondere solche in denen Y Imino ist. Verbindungen von besonderem Interesse sind 4-Ethoxy-2-hydroxy-6-methoxy-N-(p-methoxybenzyl)benzamid, 2-[(p-Anisylamino)carbonyl]-5-ethoxy-3-methoxyphenyl-dihydrogenphosphat und das Dinatriumsalz davon.

Die neuen tetra-substituierten Benzolderivate der Formel I und deren pharmazeutisch anwendbaren Salze werden erfindungsgemäß dadurch hergestellt, daß man

a) eine Verbindung der Formel

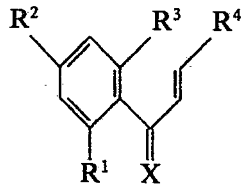

(II)

worin X, $R^1$, $R^2$, $R^3$ und $R^4$ wie in Formel I sind,

in Gegenwart eines Katalysators in einem Lösungsmittel hydriert, oder

b) ein reaktionsfähiges Derivat einer Carbonsäure der Formel

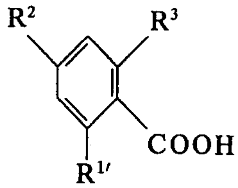

(III)

worin $R^2$ und $R^3$ wie in Formel I sind und $R^{1'}$ eine geschützte Hydroxygruppe ist, mit einer Verbindung der Formel

$$R^4-NH_2 \qquad (IV)$$

worin $R^4$ wie in Formel I ist, umsetzt und sodann die Schutzgruppe von der Hydroxygruppe entfernt, oder

c) ein Acetophenon der Formel

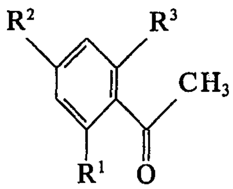

(V)

worin $R^1$, $R^2$ und $R^3$ wie in Formel I sind, mit Jod in einem tertiären Amin umsetzt und das erhaltene Salze mit einem Amin der obigen Formel IV umsetzt, oder

d) einen Ester der Formel

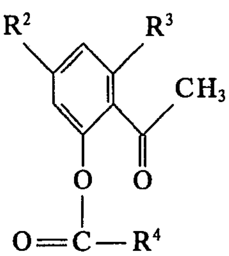

(VI)

worin $R^2$, $R^3$ und $R^4$ wie in Formel I sind, in Gegenwart einer Base in einem Lösungsmittel umlagert, oder

e) ein Keton der Formel

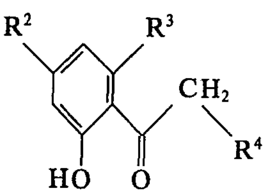

(VII)

worin $R^2$, $R^3$ und $R^4$ wie in Formel I sind, mit einem Hydroxylaminsalz in einem Lösungsmittel umsetzt, oder

f) eine Carbonylverbindung der Formel

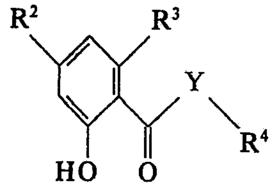

(VIII)

worin Y, $R^2$, $R^3$ und $R^4$ wie in Formel I sind, mit Phosphorpentasulfid in Gegenwart einer Base in einem Lösungsmittel umsetzt, oder

g) ein Phenol der Formel

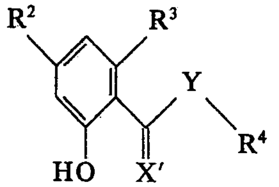

(IX)

worin Y, $R^2$, $R^3$ und $R^4$ wie in Formel I sind und X' Sauerstoff oder Schwefel ist, mit einem Acylierungsmittel in Gegenwart einer Base umsetzt, oder

h) ein Phenol der obigen Formel IX mit Phosphoroxychlorid oder Dibenzylphosphorochloridat in Gegenwart einer Base in einem Lösungsmittel umsetzt und das Reaktionsprodukt hydrolysiert bzw. hydrogenolysiert und gewünschtenfalls eine erhaltene Verbindung in ein Salz überführt, oder

i) ein Phenol der obigen Formel IX mit einem Glycosylhalogenid oder einem acylierten Glycosylhalogenid in Gegenwart eines Katalysators in einem Lösungsmittel umsetzt und gewünschtenfalls danach die Acylgruppen entfernt.

Die Hydrierung gemäß Verfahrensvariante a) kann durch Behandlung der Verbindung der Formel II mit Wasserstoff in Gegenwart eines Katalysators wie Palladiumschwarz oder Palladium/Kohle in einem Lösungsmittel wie Chloroform vorgenommen werden.

Die Umsetzung gemäß Verfahrensvariante b) kann durch Behandlung eines reaktionsfähigen Derivats einer Carbonsäure der Formel III mit einem Amin der Formel IV, gefolgt von Entfernung der Schutzgruppe von der Hydroxygruppe vorgenommen werden. Bevorzugte Beispiele von reaktionsfähigen Derivaten sind Acylhalogenide wie Acylchloride oder -bromide und aktivierte Ester wie der N-Hydroxysuccinimidester oder der p-Nitrophenylester. Die Entfernung der Schutzgruppe aus der geschützten Hydroxygruppe kann mit an sich bekannten Methoden vorgenommen werden.

Die Umsetzung gemäß Verfahrensvariante c) ist eine neuartige Reaktion. Dabei wird ein Acetophenon der Formel V mit Jod in einem tertiären Amin, wie Pyridin oder Lutidin bei erhöhter Temperatur umgesetzt, worauf das erhaltene Salz mit einem Amin der Formel IV umgesetzt wird.

Die Basen-katalysierte Umlagerung gemäß Verfahrensvariante d) kann dadurch vorgenommen werden, daß man einen Ester der Formel VI in Gegenwart einer Base wie Kaliumcarbonat, Natriumhydrid oder Natriumamid in einem Lösungsmittel wie Benzol, Toluol, Tetrahydrofuran oder Dioxan behandelt.

Die Oxidierung gemäß Verfahrensvariante e) kann durch Umsetzung des Ketons der Formel VII mit einem Hydroxylaminsalz in einem Lösungsmittel wie Dimethylsulfoxid vorgenommen werden.

Die Umsetzung gemäß Verfahrensvariante f) kann durch Umsetzung einer Carbonylverbindung der Formel VIII mit Phosphorpentasulfid in Gegenwart einer Base wie Triethylamin in einem Lösungsmittel wie Schwefelkohlenstoff vorgenommen werden.

Die Acylierung der Hydroxygruppe in einem Phenol der Formel IX gemäß Verfahrensvariante g) kann in an sich bekannter Weise durch Behandlung mit einem Acylierungsmittel wie Acetanhydrid, Ethoxycarbonylchlorid, Stearinsäureanhydrid oder Benzoylchlorid in Gegenwart einer Base wie Natriumacetat, Pyridin, Triethylamin oder 4-(Dimethylamino)pyridin vorgenommen werden.

Die Phosphylierung der Hydroxygruppe in einem Phenol der Formel IX gemäß Verfahrensvariante h) kann in an sich bekannter Weise durch Behandlung mit Phosphoroxychlorid oder Dibenzylphosphorochloridat in Gegenwart einer Base wie Triethylamin oder N,N-Diisopropylamin in einem Lösungsmittel wie Benzol, Toluol oder Triethylamin vorgenommen werden. Wenn Phosphoroxychlorid benützt wird, wird die erhaltene Verbindung hydrolysiert. Wenn Dibenzylphosphorochloriat verwendet wird, wird die erhaltene Verbindung hydrogenolysiert. Das so erhaltene Phosphat einer Verbindung der Formel IX kann in an sich bekannter Weise in ein Salz umgewandelt werden.

Die Glycosylierung der Hydroxygruppe eines Phenols der Formel IX gemäß Verfahrensvariante i) kann in an sich bekannter Weise durch Behandlung mit einem Glycosylhalogenid, in dem die Hydroxygruppen geschützt sind, beispielsweise durch Schutzgruppen wie Acetyl oder Benzyl. Bevorzugte Glucosylreste sind Glucosyl, Mannosyl und Glucosaminyl.

Die Verbindungen der Formel I zeigen antivirale Aktivität, insbesondere hemmen sie die Replikation menschlicher Rhinoviren in HeLa Zellkulturen in einer Konzentration von 0,001 bis 2,7 µg/ml.

Die vorliegende Erfindung betrifft weiterhin antivirale Mittel die eine Verbindung der Formel I oder ein pharmazeutisch annehmbares Salz davon enthalten. Die Verbindungen der Formel I sind besonders wirksam gegen gewisse Viren der Picornagruppe. Die folgenden Verbindungen haben eine besonders starke antivirale Aktivität:

1-(4-Ethoxy-2-hydroxy-6-methoxyphenyl)-3-(4-methoxyphenyl)-1-propanon,
5-Ethoxy-3-methoxy-2-[3-(p-methoxyphenyl)-propionyl]-phenyl-acetat,
4-Ethoxy-2-hydroxy-6-methoxy-N-(p-methoxybenzyl)-benzamid,
2-Hydroxy-4,6-dimethoxy-N-[p-(methylthio)benzyl]-benzamid,
2-{[p-(Allyloxy)benzyl]amino]carbonyl}-3,5-dimethoxyphenyl-benzoat,
1-(4-Ethoxy-2-hydroxy-6-methoxyphenyl)-3-(4-methoxyphenyl)-1-propanthion,
5-Ethoxy-3-methoxy-2-[3-(4-methoxyphenyl)propionyl]-phenylethylcarbonat,
2-[(p-Anisylamino)carbonyl]-5-ethoxy-3-methoxyphenyldihydrogen-phosphat,
Dinatrium 2-[(p-Anisylamino)carbonyl]-5-ethoxy-3-methoxyphenyl-phosphat,
2-Hydroxy-6-methoxy-4-propoxy-N-(p-methoxybenzyl)-benzamid,
4-(Allyloxy)-2-hydroxy-6-methoxy-N-(p-methoxybenzyl)-benzamid,
2-Hydroxy-6-methoxy-4-(3-methyl-2-butenyloxy)-N-(p-methoxybenzyl)benzamid und
Dinatrium 2-[(p-Anisylamino)carbonyl]-3-methoxy-5-propoxyphenyl-phosphat.

## Antivirale Aktivität

Eine Suspension von HeLa-Zellen ($6 \times 10^4$) wurde mit Rhinovirus HGP ($3 \times 10^3$ Plaque-bildende Einheiten, PFU) gemischt und auf eine Mikrotestplatte gebracht, die die zu testenden Verbindungen in zunehmender Verdünnung enthielten. Die Zellen wurden dann in Eagles Minimalnährlösung, die 2% Kalbsserum, 1% Tryptosephosphatbrühe, 100 µg/ml Streptomycin und 20 Einheiten/ml Penicillin G enthielt, kultiviert. Der virale zytopathogenische Effekt (C.P.E.) und die Cytotoxizität wurden mikroskopisch nach 2 Tagen Kultivierung bei 33°C beobachtet. Die antivirale Aktivität ($IC_{50}$) der Testbedingungen ist diejenige Konzentration, bei der C.P.E. um 50% gegenüber der Kontrollkultur gehemmt war. Die Cytotoxizität ist als Minimalkonzentration angegeben, bei der toxische Symtome beobachtet wurden (cytotoxische Dosis).

Die Resultate sind in Tabelle 1 angegeben und zeigen, daß die erfindungsgemäßen Verbindungen eine Anti-Rhinovirus-Aktivität bei Konzentrationen entwickelten, die 10- bis 10 000mal niedriger als die cytotoxischen Dosen waren.

Weiterhin sind die antiviralen Spektren von 1-(4-Ethoxy-2-hydroxy-6-methoxyphenyl)-3-(4-methoxyphenyl)-1-propanon (Verbindung A) und 4-Ethoxy-2-hydroxy-6-methoxy-N-(p-methoxybenzyl)benzamid (Verbindung B) gegen verschiedene Serotypen von Rhinoviren in Tabelle 2 angegeben.

Tabelle 1

| Verbindung | $IC_{50}$ (µg/ml) | Cytotoxizität (µg/ml) |
|---|---|---|
| 1-(2-Hydroxy-4,6-dimethoxyphenyl)-3-[3,4-(methylenedioxy)phenyl]-1-propanon | 0,03—0,1 | >8 |
| 1-(2-Hydroxy-4,6-dimethoxyphenyl)-3-[4-methylthio)-phenyl]-1-propanon | 0,01—0,03 | 8 |
| 1-(2-Hydroxy-4,6-dimethoxyphenyl)-3-(4-methylphenyl)-1-propanon | 0,01—0,03 | >8 |
| 1-(4-Ethoxy-2-hydroxy-6-methoxyphenyl)-3-(4-methoxyphenyl)-4-propanon | 0,002 | >8 |
| 5-Ethoxy-3-methoxy-2-[3-(p-methoxyphenyl)propionyl]-phenyl-dihydrogenphosphat | 0,3—0,8 | >10 |

Tabelle 1 (Fortsetzung)

| Verbindung | $IC_{50}$ (μg/ml) | Cytotoxizität (μg/ml) |
|---|---|---|
| 5-Ethoxy-3-methoxy-2-[3-(p-methoxyphenyl)propionyl]-phenylacetat | 0,001 | >8 |
| 1-(2-Hydroxy-4,6-dimethoxyphenyl)-3-(4-methoxyphenyl)-1-propanon-oxim (Z-isomer) | 0,03 | >10 |
| 1-(2-Hydroxy-4,6-dimethoxyphenyl)-3-(4-methoxyphenyl)-1-propanon-oxim (E-isomer) | 0,3—0,9 | >10 |
| 2-Hydroxy-4,6-dimethoxy-N-(p-methoxybenzyl)-benzamid | 0,002 | 8 |
| 3-(4-Chlorophenyl)-1-(2-hydroxy-4,6-dimethoxy-phenyl)-1-propanon | 0,1 | 10 |
| 1-(2-Hydroxy-4,6-dimethoxyphenyl)-3-(5-methyl-2-furyl)-1-propanon | 0,9 | >8 |
| 1-(2-Ethoxy-6-hydroxy-4-methoxyphenyl)-3-(4-methoxyphenyl)-1-propan | 0,01—0,03 | 0,9 |
| 1-(2-Hydroxy-4,6-dimethoxyphenyl)-3-(4-methoxyphenyl)-1,3-propandion | 0,01—0,03 | 8 |
| 1-(2-Hydroxy-6-methoxy-4-propoxyphenyl)-3-(4-methoxyphenyl)-1-propanon | 0,004—0,01 | 2,7 |
| 1-(2-Hydroxy-4-isopropoxy-6-methoxyphenyl)-3-(4-methoxy-phenyl)-1-propanon | 0,03—0,1 | 8 |
| 4-Ethoxy-2-hydroxy-6-methoxy-N-(p-methoxybenzyl)benzamid | 0,002 | >10 |
| 1-(2-Hydroxy-4,6-dimethoxyphenyl)-3-(tetrahydro-5-methyl-2-furyl)-1-propanon | 0,9 | >8 |
| 2-Hydroxy-4,6-dimethoxy-N-(4-methoxyphenyl)benzamid | 0,03—0,1 | >10 |
| N-Benzyl-2-hydroxy-4,6-dimethoxybenzamid | 0,03—0,1 | >8 |
| 2-Hydroxy-4,6-dimethoxy-N-[m,p-(methylenedioxy)-benzyl]benzamid | 0,003—0,01 | >8 |
| 2-Hydroxy-4,6-dimethoxy-N-(m-methoxybenzyl)-benzamid | 0,002 | >8 |
| 2-Hydroxy-4,6-dimethoxy-N-(p-methylbenzyl)-benzamid | 0,01—0,03 | 8 |
| N-(p-Chlorobenzyl)-2-hydroxy-4,6-dimethoxybenzamid | 0,01—0,03 | >2,4 |
| N-Furfuryl-2-hydroxy-4,6-dimethoxybenzamid | 0,03—0,1 | >8 |
| 2-Hydroxy-4,6-dimethoxy-N-methylbenzamid | 0,9 | >8 |
| 2-Hydroxy-N-(m-hydroxy-p-methoxybenzyl)-4,6-dimeth-oxybenzamid | 0,1—0,3 | 2,7 |
| N-(m,p-Dimethoxybenzyl)-2-hydroxy-4,6-dimethoxybenzamid | 0,3 | 8 |
| 2-Hydroxy-4,6-dimethoxy-N-[(4-pyridyl)methyl]-benzamid | 0,9 | 8 |

Tabelle 1 (Fortsetzung)

| Verbindung | $IC_{50}$ (µg/ml) | Cytotoxizität (µg/ml) |
|---|---|---|
| N-(p-Butoxybenzyl)-2-hydroxy-4,6-dimethoxybenzamid | 0,3 | 8 |
| 2-Hydroxy-N-(p-hydroxybenzyl)-4,6-dimethoxybenzamid | 0,3 | >8 |
| N-[p-Dimethylamino)benzyl]-2-hydroxy-4,6-dimethoxybenzamid | 0,01 | >8 |
| 2-Hydroxy-4,6-dimethoxy-N-[p-(methylthio)benzyl]-benzamid | 0,002 | >8 |
| N-[p-(Benzyloxy)benzyl]-2-hydroxy-4,6-dimethoxybenzamid | 0,3—0,9 | >8 |
| 2-Hydroxy-4,6-dimethoxy-N-(2-thenyl)benzamid | 0,03 | 8 |
| N-[p-(Allyloxy)benzyl]-2-hydroxy-4,6-dimethoxybenzamid | 0,01 | 8 |
| 2-Hydroxy-4,6-dimethoxy-N-(tetrahydro-2-thenyl)-benzamid | 0,004—0,01 | 8 |
| 2-Hydroxy-4,6-dimethoxy-N-[(2-pyrrolyl)methyl]-benzamid | 2,7 | >8 |
| 2-[(p-Anisylamino)-carbonyl]-5-ethoxy-3-methoxyphenyl-acetat | 0,3 | >8 |
| 2-[[[p-(Allyloxy)benzyl]-amino]carbonyl]-3,5-dimethoxy-phenyl-benzoat | 0,002 | 2,7 |
| 2-{[[p-(allyloxy)benzyl]-amino]carbonyl}-3,5-dimethoxy-phenyl-octadecanoat | 0,3 | >8 |
| 1-(4-Ethoxy-2-hydroxy-6-methoxyphenyl)-3-(4-methoxy-phenyl)1-propanthion | 0,002 | 2,7 |
| 4-Ethoxy-2-hydroxy-6-methoxy-N-(p-methoxybenzyl)thiobenzamid | 0,01 | >8 |
| 1-[2-Hydroxy-6-methoxy-4-(methylthio)phenyl-3-(4-meth-oxyphenyl)-1-propanon | 0,1 | 1 |
| 5-Ethoxy-3-methoxy-2-[3-(4-methoxyphenyl)propionyl]-phenyl-ethylcarbonat | 0,002 | 8 |
| 5-Ethoxy-3-methoxy-2-[3-(4-methoxyphenyl)propionyl]-phenyl-octadecanoat | 0,9 | >8 |
| 5-Ethoxy-3-methoxy-2-[3-(4-methoxyphenyl)-propionyl]-phenyl-benzoat | 0,01 | >8 |
| 2-[(p-Anisylamino)carbonyl]-3,5-dimethoxyphenyl-ethyl-carbonat | 0,1 | >8 |
| 2-[(p-Anisylamino)carbonyl]-3,5-dimethoxyphenyl-benzoat | 0,03 | >8 |
| 2-[(p-Anisylamino)carbonyl]-3,5-dimethoxyphenyl-octadecanoat | 0,3—0,9 | >8 |
| 2-[(p-Anisylamino)carbonyl]-3,5-dimethoxyphenyl tetra-O-acetyl-β-D-glucopyranosid | 2,7 | >10 |
| 2-[(p-Anisylamino)carbonyl]-3,5-dimethoxyphenyl-β-D-glucopyranosid | 2,7 | >10 |
| 5-Ethoxy-3-methoxy-2-[3-(4-methoxyphenyl)propionyl]-phenyl-β-D-glucopyranosid | 2,7 | >10 |

Tabelle 1 (Fortsetzung)

| Verbindung | $IC_{50}$ (µg/ml) | Cytotoxizität (µg/ml) |
|---|---|---|
| Disodium 5-ethoxy-3-methoxy-2-[3-(4-methoxyphenyl)-propionyl]phenyl-phosphat | 0,03—0,1 | >10 |
| 2-[(p-Anisylamino)carbonyl]-3,5-dimethoxyphenyl-dihydrogen-phosphat | 0,006—0,01 | >8 |
| 2-[(p-Anisylamino)carbonyl]-5-ethoxy-3-methoxyphenyl-dihydrogenphosphat | 0,01—0,03 | >8 |
| Disodium 2-[(p-anisylamino)-carbonyl]-5-ethoxy-3-meth-oxyphenyl-phosphat | 0,01—0,03 | >8 |
| 2-Hydroxy-6-methoxy-4-propoxy-N-(p-methoxy-benzyl)benzamid | 0,001—0,003 | >20 |
| 4-(Allyloxy)-2-hydroxy-6-methoxy-N-(p-methoxy-benzyl)benzamid | 0,002 | >8 |
| 2-Hydroxy-6-methoxy-4-(3-methyl-2-butenyloxy)-N-(p-methoxybenzyl)-benzamid | 0,001—0,002 | >8 |
| Disodium 2-[(p-anisylamino)-carbonyl]-3-methoxy-5-propoxy-phenyl-phosphat | 0,01—0,02 | >200 |

Tabelle 2

| Virus-Stamm | $IC_{50}$ (µg/ml) Verbindung A | Verbindung B |
|---|---|---|
| Rhinovirus 1A | 0,03—0,09 | 0,009—0,027 |
| Rhinovirus 1B | 0,03—0,09 | 0,003—0,009 |
| Rhinovirus 2 | 0,001 | 0,001 |
| Rhinovirus 9 | 0,012—0,037 | 0,003 |
| Rhinovirus 14 | 0,1—0,3 | 0,009—0,027 |
| Rhinovirus 16 | — | 0,003 |
| Rhinovirus 21 | <0,001 | <0,001 |
| Rhinovirus 23 | 0,004 | — |
| Rhinovirus 24 | 0,01—0,03 | — |
| Rhinovirus 25 | 0,01—0,09 | — |
| Rhinovirus 30 | — | 0,001—0,003 |
| Rhinovirus 31 | 0,03—0,09 | 0,081—0,24 |
| Rhinovirus 32 | — | 0,003—0,009 |
| Rhinovirus 36 | — | 0,009—0,027 |
| Rhinovirus 39 | 0,012—0,037 | 0,003 |
| Rhinovirus 44 | — | 0,003 |

Tabelle 2 (Fortsetzung)

| Virus-Stamm | IC50 (µg/ml) Verbindung A | Verbindung B |
|---|---|---|
| Rhinovirus 46 | 0,037–0,11<br>— | |
| Rhinovirus 47 | —<br>0,003 | |
| Rhinovirus 50 | 0,004–0,012<br>0,01 | |
| Rhinovirus 55 | —<br>0,009 | |

Die cytotoxischen Dosen, die HeLa-Zellwachstum hemmen, sind 60 µg/ml für die Verbindung A und 40 µg/ml für die Verbindung B.

Wie oben erwähnt, können die Verbindungen der Formel I und deren pharmazeutisch anwendbare Salze als Heilmittel gegen Viruserkrankungen, insbesondere bei Erkältungen in Form pharmazeutischer Präparate verwendet werden.

Die pharmazeutischen Präparate enthalten mindestens eine der erwähnten antiviralen Verbindungen oder deren pharmazeutisch anwendbaren Salze zusammen mit einem verträglichen pharmazeutischen Träger. Sie können auch andere pharmazeutische aktive Verbindungen wie fiebersenkende Mittel, schmerzstillende Mittel, antiinflammatorische Mittel, Antihistamine oder Interferon-Induktoren enthalten. Die pharmazeutischen Präparate können in fester Form für orale Verabreichung vorliegen, z. B. als Tabletten, Kapseln, Pillen, Pulver oder Granulate, in flüssiger Form zur nasalen oder oralen Verabreichung, z. B. als Lösungen, Suspensionen oder Sirupe, für parenterale Verabreichung als sterile Lösungen, Suspensionen oder Emulsionen oder für topische Verabreichung in Form von Lösungen, Emulsionen, mikronisierten Pulvern, Salben sowie als Gurgelwässer oder Aerosole.

Die Dosierungen für die Behandlung hängen von der Verabreichungsart, dem Alter, Gewicht und des Zustandes des Patienten und der zu behandelnden Krankheit ab. Im allgemeinen empfiehlt sich für Erwachsene bei Erkältungen eine Dosis von 100–200 mg drei- bis sechsmal täglich bei oraler Behandlung und etwa 0,1 bis 100 µg/cm$^2$ drei- bis sechsmal täglich im Falle der topischen Verabreichung.

Die folgenden Beispiele erläutern die Erfindung weiter.

## Beispiel 1

Eine Lösung von 328 mg 4'-Ethoxy-2'-hydroxy-4,6'-dimethoxychalcon in 10 ml Chloroform wurde in Gegenwart von 30 mg 10% Palladium/Kohle bei Raumtemperatur unter Atmosphärendruck 3 Stunden hydriert. Der Katalysator wurde abfiltriert und mit 30 ml Chloroform gewaschen, das Filtrat in die Waschwässer vereinigt und unter vermindertem Druck eingedampft. Man erhielt einen kristallinen Rückstand, der aus Methanol umkristallisiert 302 mg 1-(4-Ethoxy-2-hydroxy-6-methoxyphenyl)-3-(4-methoxyphenyl)-1-propanon in Form farbloser Nadeln vom Schmelzpunkt 100,5–101°C lieferte.

## Beispiel 2

In Analogie zu Beispiel 1 wurden die in der nachstehenden Tabelle 3 angeführten Verbindungen der Formel I aus den Chalconen erhalten.

Tabelle 3

| Chalcone | Verbindungen der Formel I |
|---|---|
| 2′-Hydroxy-4,4′,6′-trimethoxychalcon | 1(2-Hydroxy-4,6-dimethoxyphenyl)-3-(4-methoxyphenyl)-1-propanon; Schmelzpunkt 110° C (Methanol) |
| 2′-Hydroxy-4′-6′-dimethoxy-4-(methylthio)-chalcon | 1-(2-Hydroxy-4,6-dimethoxyphenyl)-3-[4-(methylthio)-phenyl]-1-propanon; Schmelzpunkt 84° —85° C (Methanol) |
| 2′-Hydroxy-4′,6′-dimethoxy-3,4-(methylene-dioxy)-chalcon | 1-(2-Hydroxy-4,6-dimethoxyphenyl)-3-[3,4-(methylendioxy)-phenyl]-1-propanon; Schmelzpunkt 124,5° C (Methanol) |
| 2′-Hydroxy-4′,6′-dimethoxy-4-methyl-chalcon | 1-(2-Hydroxy-4,6-dimethoxyphenyl)-3-(4-methylphenyl)-1-propanon: Schmelzpunkt 128° —129° C (Methanol) |
| 4-Chloro-2′-hydroxy-4′,6′-dimethoxy-chalcon | 3-(4-Chlorophenyl)-1-(2-hydroxy-4,6-dimethoxyphenyl)-1-propanon; Schmelzpunkt 104,5° C (Benzol/Hexan) |
| 2′-Ethoxy-6′-hydroxy-4,4′-dimethoxy-chalcon | 1-(2-Ethoxy-6-hydroxy-4-methoxyphenyl)-3-(4-methoxyphenyl)-1-propanon; Schmelzpunkt 108° —109° C (Benzol/Hexan) |

## Beispiel 3

Eine Lösung von 400 mg 2′-Hydroxy-4′,6′-dimethoxy-3-(5-methyl-2-furyl)acrylophenon wurde in 25 ml Chloroform in Gegenwart von 40 mg 10% Palladium/Kohle bei Raumtemperatur unter Atmosphärendruck 4 Stunden hydriert. Nach Abfiltrieren des Katalysators wurde das Filtrat zu einem öligen Rückstand eingeengt, der in einer kleinen Menge Benzol gelöst wurde. Die Lösung wurde auf eine Silicagelsäule gegeben und die Säule mit Hexan/Äthylacetat (5 : 1 Volumenteile) eluiert, wobei zwei Fraktionen, A (Rf 0,29) und B (Rf 0,16) erhalten wurden, wobei die Elution durch Dünnschichtchromatographie auf Silicagelplatten mit Cyclohexanon/Äthylacetat (4 : 1 Volumenteile) verfolgt wurde. Entfernung des Lösungsmittels aus der Fraktion A und Umkristallisation aus Benzol/Hexan lieferte 192 mg 1-(2-Hydroxy-4,6-dimethoxyphenyl)-3-(5-methyl-2-furyl)-1-propan in Form farbloser Kirstalle vom Schmelzpunkt 92—93° C.

Ähnliche Aufarbeitung der Fraktion B lieferte 25 mg 1-(2-Hydroxy-4,6-dimethoxyphenyl)-3-(tetrahydro-5-methyl-23-furyl)-1-propanon in Form farbloser Nadeln vom Schmelzpunkt 67,5° C (aus Petroläther).

## Beispiel 4

85 mg 2′-Hydroxy-4,6′-dimethoxy-4′-propoxychalcon wurden in Analogie zu dem Verfahren in Beispiel 1 hydriert. Man erhielt 69 mg 1-(2-Hydroxy-6-methoxy-4-propoxyphenyl)-3-(4-methoxyphenyl)-1-propanon in Form farbloser Nadeln vom Schmelzpunkt 85—86° C.

Das 2′-Hydroxy-4,6′-dimethoxy-4′-propoxychalcon wurde wie folgt hergestellt:

Ein Gemisch von 182 mg 2′,4′-Dihydroxy-6′-methoxyacetophenon, 187 mg Propyljodid und 276 mg wasserfreies Kaliumcarbonat wurden in 5 ml Aceton 16 Stunden zum Rückfluß erhitzt. Nach Abkühlen wurde das Gemisch mit 30 ml Wasser verdünnt und dreimal mit je 30 ml Dichlormethan extrahiert. Die vereinigten Extrakte wurden mit Wasser gewaschen, über Natriumsulfat getrocknet und eingedampft und lieferten rohes 2′-Hydroxy-6′-methoxy-4′-propoxyacetophenon als hellgelbes Öl.

Dieses Öl wurde in 5 ml Ethanol gelöst, das 120 mg p-Methoxybenzaldeyd enthielt, und 4 ml 15%ige wäßrige Natronlauge wurden zugesetzt. Nach 2tägigem Rühren bei Raumtemperatur wurde das Gemisch durch Zusatz von Salzsäure auf pH 5—6 gestellt und mit dreimal 30 ml Äthylacetat extrahiert. Die Extrakte wurden mit Wasser gewaschen, über Natriumsulfat getrocknet und zu einem öligen Rückstand eingedampft. Umkristallisation des Rückstandes aus Methanol lieferte 110 mg 2′-Hydroxy-4,6′-dimethoxy-4′-propoxychalcon in Form gelber Nadeln vom Schmelzpunkt 95—96° C.

Beispiel 5

In Analogie zu Beispiel 1 wurde aus 2'-Hydroxy-4'-isopropoxy-4,6'-dimethoxychalcon das 1-(2-Hydroxy-4-isopropoxy-6-methoxyphenyl)-3-(4-methoxyphenyl)-1-propanon vom Schmelzpunkt 96,5°C (aus Methanol) erhalten.

Das Ausgangsmaterial wurde wie in Beispiel 4 beschrieben hergestellt, wobei Isopropyljodid statt Propyljodid verwendet wurde.

Beispiel 6

120 mg 2'-Hydroxy-4,6'-dimethoxy-4'-(methylthio)-chalcon wurden in 10 ml Chloroform gelöst. Die Lösung wurde 33 Stunden unter Atmosphärendruck bei Raumtemperatur in Gegenwart von 180 mg 10%igen Palladium/Kohle-Katalysator hydriert. Nach Abfiltrieren des Katalysators wurde das Filtrat zu einem hellgelben Rückstand eingedampft, der aus Methanol umkristallisiert wurde. Man erhielt 103 mg 1-[2-Hydroxy-6-methoxy-4-(methylthio)-phenyl]-3-(4-methoxyphenyl)-1-propanon als crèmefarbige Nadeln vom Schmelzpunkt 127—127,5°C.

Das Ausgangsmaterial wurde wie folgt hergestellt:

Zu einer Lösung von 120 mg 2'-Hydroxy-6'-methoxy-4'-(methylthio)acetophenon und 92 mg p-Methoxybenzaldehyd in 6 ml Ethanol wurden 4 ml 15%ige Natronlauge gegeben. Nach 24 Stunden Rühren bei Raumtemperatur wurde das Gemisch mit 10 ml Wasser verdünnt und mit 1N Salzsäure angesäuert. Der erhaltene Niederschlag wurde abfiltriert, mit wenig 50%igen Methanol gewaschen und aus Methanol umkristallisiert und lieferte 144 mg 2'-Hydroxy-4,6'-dimethoxy-4'-(methylthio)chalcon in Form gelber Nadeln vom Schmelzpunkt 145—147°C.

Beispiel 7

1 g 2-(Benzyloxy)-4,6-dimethoxybenzoesäure wurde in 5 ml Thionylchlorid gelöst. Die Lösung wurde 1 Stunde bei Raumtemperatur gerührt. Entfernen des überschüssigen Thionylchlorids durch wiederholtes Abdampfen mit Benzol lieferte ein Öl, das in 5 ml Benzol gelöst wurde. Die Lösung wurde tropfenweise zu einer eiskalten Lösung von 1,8 ml p-Methoxybenzylamin in 5 ml Benzol gegeben. Nach 17 Stunden Rühren bei Raumtemperatur wurde das Gemisch mit 50 ml Äthylacetat verdünnt, nacheinander mit verdünnter Salzsäure und Wasser gewaschen, über Natriumsulfat getrocknet und eingedampft. Man erhielt 1,66 g eines Öls. Dieses Öl wurde in 2 ml Benzol gelöst und die Lösung wurde auf eine Silicagelsäule (60 g in Hexan)/Äthylacetat (1 : 1 Volumenteile) eluiert. Entfernung des Lösungsmittels aus dem Eluat und anschließende Umkristallisation aus Äthylacetat lieferte 700 mg 1-(Benzyloxy)-4,6-dimethoxy-N-(p-methoxybenzyl)benzamid in Form farbloser Nadeln vom Schmelzpunkt 123—124°C.

Eine Lösung von 700 mg dieses Benzamids in 20 ml Chloroform wurde $4^1/_2$ Stunden in Gegenwart von 140 mg 10%igen Palladium/Kohle bei Raumtemperatur unter Atmosphärendruck hydriert. Entfernung des Katalysators und Eindampfen des Filtrats lieferte einen kristallinen Rückstand, der aus Methanol umkristallisiert 475 mg 2-Hydroxy-4,6-dimethoxy-N-(p-methoxybenzyl)benzamid als farblose Nadeln vom Schmelzpunkt 108—109°C lieferte.

Die 2-(Benzyloxy)-4,6-dimethoxybenzoesäure wurde wie folgt hergestellt:

Zu einer gerührten Lösung von 39,75 g 2-Hydroxy-4,6-dimethoxybenzoesäure-methylester und 207 g wasserfreies Kaliumcarbonat in 2000 ml Aceton wurden tropfenweise eine Lösung von 22,3 ml Benzylbromid in 300 ml Aceton gegeben. Das Gemisch wurde 78 Stunden bei Raumtemperatur gerührt, abfiltriert und das Filtrat eingedampft. Man erhielt ein Öl, das in 500 ml Chloroform gelöst wurde. Die Lösung wurde mit 400 ml Wasser gewaschen, über Natriumsulfat getrocknet und eingedampft und lieferte 78,7 g rohes 2-(Benzyloxy)-4,6-dimethoxybenzoesäuremethylester als farbloses Öl.

78,7 g dieses rohen Esters wurden in 2200 ml Dioxan/Methanol (4 : 1 Volumenteile) gelöst. Die Lösung wurde mit 900 ml 2,4 N Natronlauge versetzt, 16 Stunden zum Rückfluß erhitzt, gekühlt und dann mit Salzsäure angesäuert.

Der erhaltene Niederschlag wurde abfiltriert und mit Äthylacetat gewaschen und lieferte 48 g 2-(Benzyloxy)-4,6-dimethoxybenzoesäure als farblose Nadeln vom Schmelzpunkt 167—168°C.

Beispiel 8

In Analogie zu dem Verfahren von Beispiel 7 wurden die in der nachstehenden Tabelle 4 aufgeführten Verbindungen der Formel I aus den in der Tabelle 4 genannten Aminen erhalten.

Tabelle 4

| Amine | Verbindungen der Formel I |
|---|---|
| p-Methoxyanilin | 2-Hydroxy-4,6-dimethoxy-N-(4-methoxyphenyl) benzamid; Schmelzpunkt 131° — 132° C (aus Methanol) |
| Benzylamin | N-Benzyl-2-hydroxy-4,6-dimethoxybenzamid; Schmelzpunkt 96° C (Methanol) |
| m,p-(Methylenedioxy)-benzylamin | 2-Hydroxy-4,6-dimethoxy-N-(m,p-methylenedioxy-benzyl)benzamid; Schmelzpunkt 116° — 117° C (Methanol) |
| p-Methylbenzylamin | 2-Hydroxy-4,6-dimethoxy-N-(p-methylbenzyl)-benzamid; Schmelzpunkt 100—110,5° C (Methanol) |
| p-Chlorobenzylamin | N-(p-Chlorobenzyl)-2-hydroxy-4,6-dimethoxy-benzamid; Schmelzpunkt 131° — 132° C (Methanol) |
| 2-(Aminomethyl)furan | N-Furfuryl-2-hydroxy-4,6-dimethoxybenzamid; Schmelzpunkt 74° — 75° C (Methanol) |
| Methylamin | 2-Hydroxy-4,6-dimethoxy-N-methylbenzamid; Schmelzpunkt 144° — 145,5° C (Methanol) |
| m-(Benzyloxy)-p-methoxybenzylamin | 2-Hydroxy-N-(m-hydroxy-p-methoxybenzyl)-4,6-dimethoxybenzamid; Schmelzpunkt 144° — 145° C (Methanol) |
| m,p-Dimethoxybenzylamin | 2-Hydroxy-4,6-dimethoxy-N-(m,p-dimethoxybenzyl)-benzamid; Schmelzpunkt 115° — 116° C (Methanol) |
| p-Butoxybenzylamin | N-(p-Butoxybenzyl)-2-hydroxy-4,6-dimethoxy-benzamid; Schmelzpunkt 88° — 89° C (Methanol) |
| p-Hydroxybenzylamin | 2-Hydroxy-N-(p-hydroxybenzyl)-4,6-dimeth-oxybenzamid; Schmelzpunkt 170° — 171° C (Methanol) |
| p-(Dimethylamino)-benzylamin | N-[p-(Dimethylamino)benzyl]-2-hydroxy-4,6-dimethoxybenzamid; Schmelzpunkt 79° — 82° C (Methanol) |
| p-(Methylthio)-benzylamin | 2-Hydroxy-4,6-dimethoxy-N-[p-(methylthio)-benzyl]benzamid; Schmelzpunkt 119° — 120° C (Methanol) |
| p-(Benzyloxy)-benzylamin | N-[p-(Benzyloxy)benzyl]-2-hydroxy-4,6-dimeth-oxybenzamid; Schmelzpunkt 109° — 110° C (Methanol) |
| 2-Thenylamin | 2-Hydroxy-4,6-dimethoxy-N-(2-thenyl)benzamid; Schmelzpunkt 60° C (Methanol) |
| Tetrahydro-2-thenylamin | 2-Hydroxy-4,6-dimethoxy-N-(tetrahydro-2-thenyl)benzamid; Schmelzpunkt 81° — 82° C (Methanol) |

## Beispiel 9

Zu einer gekühlten Suspension von 1 g 2-(Benzyloxy)-4,6-dimethoxybenzoesäure und 400 mg N-Hydroxysuccinimid in 20 ml Dioxan wurden 858 mg Dicyclohexylcarbodiimid gegeben. Das Gemisch wurde 19 Stunden bei Raumtemperatur gerührt und dann filtriert. Entfernung des Lösungsmittels aus dem Filtrat lieferte ein Öl, das an 50 g Silicagel chromatographiert wurde. Als Elutionsmittel wurde Hexan/Äthylacetat (4 : 1 Volumenteile) verwendet. Das Eluat wurde zu einem Sirup eingedampft, der beim Stehen an einem kühlen Ort sich zu einer kristallinen Masse verfestigte.

225 mg dieses Feststoffes wurden in 5 ml Dimethylformamid gelöst. Die Lösung wurde mit 88 mg p-Methoxybenzylamin versetzt und 18 Stunden bei Raumtemperatur gerührt. Danach wurde das Gemisch mit 20 ml 1N Salzsäure versetzt und zweimal mit je 50 ml Äthylacetat extrahiert. Die Extrakte wurden mit Wasser gewaschen, über Natriumsulfat getrocknet und zu einem kristallinen Rückstand eingedampft. Umkristallisation aus Äthylacetat/Hexan lieferte 228 mg 2-(Benzyloxy)-4,6-dimethoxy-N-(p-methoxybenzyl)benzamid in Form farbloser Nadeln vom Schmelzpunkt 123—124° C.

Entfernung der Benzylgruppe aus dem Benzyamid durch Hydrogenolyse gemäß dem Verfahren von Beispiel 7 lieferte 2-Hydroxy-4,6-dimethoxy-N-(p-methoxybenzyl)benzamid vom Schmelzpunkt 108—109° C.

## Beispiel 10

Zu einer Lösung von 980 mg 2'-Hydroxy-4',6'-dimethoxyacetophenon in 2,0 ml Pyridin wurden 1,27 g Jod gegeben. Das Gemisch wurde 1 Stunde auf 100° erhitzt, wobei sich eine feste Masse bildete. Nach Kühlen wurde der Feststoff nacheinander mit 50 ml Äther und etwas kaltem Wasser gewaschen und dann unter vermindertem Druck getrocknet. Man erhielt 2 g rohes 1-[(2-Hydroxy-4,6-dimethoxybenzol)methyl]pyridiniumjodid in Form eines braunen Pulvers.

1 g dieses Pyridiniumsalzes wurde zu 514 mg p-Methoxybenzylamin gegeben und das Gemisch wurde 72 Stunden auf 80°C erwärmt. Nach Abkühlen wurde das Gemisch in 20 ml 1N Salzsäure gegossen. Die erhaltene Suspension wurde mit zweimal 100 ml Dichlormethan extrahiert, die Extrakte wurden mit Wasser gewaschen, über Natriumsulfat getrocknet und eingedampft. Man erhielt 945 mg eines öligen Rückstandes, der durch Chromatographie an Silicagel mit Hexan/Äthylacetat (3 : 1 Volmenteile) als Elutionsmittel gereinigt wurde. Entfernung des Lösungsmittels aus dem Eluat und anschließende Umkristallisation aus Methanol lieferte 316 mg 2-Hydroxy-4,6-dimethoxy-N-(p-methoxybenzyl)benzamid vom Schmelzpunkt 108—109° C.

## Beispiel 11

In Analogie zu Beispiel 10 wurden die in Tabelle 5 aufgeführten Verbindungen der Formel I aus den Pyridiniumsalzen der in der Tabelle 5 angeführten Amine erhalten.

Tabelle 5

| Amine | Verbindungen der Formel I |
|---|---|
| 4-(Aminomethyl)pyridin | 2-Hydroxy-4,6-dimethoxy-N-[(4-pyridyl)-methyl]benzamid; Schmelzpunkt 114—115° C (aus Äther) |
| p-(Allyloxy)benzylamin | N-[p-(Allyloxy)benzyl-2-hydroxy-4,6-dimethoxybenzamid; Schmelzpunkt 93—94° C (Methanol) |
| 2-(Aminomethyl)pyrrol | 2-Hydroxy-4,6-dimethoxy-N-(2-pyrrolyl-methyl)benzamid; Schmelzpunkt 116—117° (Äther) |

## Beispiel 12

Eine Lösung von 9,8 g 4'-Ethoxy-2'-hydroxy-6'-methoxyacetophenon und 12 g Jod in 22 ml Pyridin wurde 1 Stunde auf 100° C erwärmt. Die erhaltene feste Masse wurde gekühlt, pulverisiert und erneut 1 Stunde auf 100° C erwärmt. Nach Kühlen wurde das Gemisch filtriert und der feste Rückstand nacheinander mit 100 ml Äther und 100 ml Wasser gewaschen und dann 3 Stunden bei 60° C unter vermindertem Druck getrocknet. Man erhielt 18,2 g 1-[(4-Ethoxy-2-hydroxy-6-methoxybenzoyl)methyl]pyridiniumjodid als hellbraunen Feststoff.

15,4 g dieses Pyridniumsalzes wurden zu 10,0 ml p-Methoxybenzylamin gegeben und das Gemisch wurde 18 Stunden unter Stickstoff und unter Rühren auf 80° C erwärmt. Nach Kühlen wurde das Gemisch mit 200 ml Äthylacetat und 150 ml Wasser versetzt. Die Äthylacetatphase wurde abgetrennt und die wäßrige Phase mit 150 ml Äthylacetat extrahiert. Die vereinigten Äthylacetatlösungen wurden nacheinander mit zweimal 150 ml verdünnter Salzsäure, 150 ml wäßrigen Natriumcarbonat und Kochsalzlösung gewaschen, über Natriumsulfat getrocknet und eingedampft. Man erhielt 15 g eines Öls, das durch Chromatographie an Silicagel mit Hexan/Äthylacetat gereinigt wurde. Entfernen des Lösungsmittel aus dem Eluat und Kristallisation des Rückstandes aus Methanol lieferte 6,3 g 4-Ethoxy-2-hydroxy-6-methoxy-N-(p-methoxybenzyl)-benzamid in Form farbloser Nadeln vom Schmelzpunkt 87—88° C.

## Beispiel 13

Ein Gemisch von 336 mg 2-Acetyl-3,5-dimethoxyphenyl-4-methoxybenzoat und 735 mg wasserfreiem Kaliumcarbonat in 5 ml Toluol wurde 17 Stunden auf 100° C erwärmt, dann gekühlt und filtriert. Nach Waschen mit Benzol wurde der Filterkuchen mit 50 ml Dichlormethan und 50 ml Wasser behandelt. Die Dichlormethanphase wurde abgetrennt, über Natriumsulfat getrocknet und zu einem kristallinen Feststoff eingedampft. Umkristallisation des Rückstandes aus Methanol lieferte 77 mg 1-(2-Hydroxy-4,6-dimethoxyphenyl)-3-(4-methoxyphenyl)-1,3-propandion in Form gelber Prismen vom Schmelzpunkt 136—136,5° C.

## Beispiel 14

Zu einer gerührten Suspension von 580 mg 1-(2-Hydroxy-4,6-dimethoxyphenyl)-3-(4-methoxyphenyl)-1-propanon und 482 mg Natriumacetat in 9 ml Dimethylsulfoxid wurden 204 mg Hydroxylamin-hydrochlorid gegeben. Das Gemisch wurde 15,5 Stunden auf 80° C erwärmt, gekühlt, mit 30 ml Äthylacetat verdünnt und nacheinander mit verdünnter Salzsäure und Wasser gewaschen. Nach Trocknen über Natriumsulfat und Eindampfen erhielt man 710 mg eines gelben Öls, das in 1 ml Benzol gelöst wurde.

Diese Lösung wurde auf eine Säule von 25 g Silicagel gegeben und die Säule wurde mit Hexan/Äthylacetat (3 : 1 Volumenteile) eluiert. Dabei wurden 15-ml-Fraktionen gesammelt. Die Fraktionen 5—8 wurden vereinigt und eingedampft und lieferten 435 mg eines hellgelben öligen Rückstandes. Umkristallisation aus Äthylacetat/Hexan lieferte 301 mg des E-Isomers von 1-(2-Hydroxy-4,6-dimethoxyphenyl)-3-(4-methoxyphenyl)-1-propanon-oxim in Form farbloser Kristalle vom Schmelzpunkt 82—83,5° C.

Das entsprechende Z-Isomer wurde ebenfalls in Form farbloser Kristalle (Schmelzpunkt 102—104° C) aus den Fraktionen 20—30 nach Entfernen des Lösungsmittels und anschließende Umkristallisation aus Äthylacetat/Hexan in einer Ausbeute von 95 mg erhalten.

## Beispiel 15

Ein Gemisch von 500 mg 4-Ethoxy-2-hydroxy-6-methoxy-N-(p-methoxybenzyl)benzamid, 673 mg Phosphorpentasulfid und 259 mg Triethylamin in 10 ml Schwefelkohlenstoff wurde 3 Tage bei Raumtemperatur gerührt. Danach wurden 100 ml Äthylacetat und 100 ml Wasser zugesetzt und das Gemisch wurde geschüttelt. Die Äthylacetatphase wurde abgetrennt und die wäßrige Phase mit 100 ml Äthylacetat extrahiert. Die vereinigten Äthylacetatlösungen wurden mit Kochsalzlösung gewaschen und eingedampft. Der Rückstand wurde an Silicagel mit Benzol/Äthylacetat (10 : 1 Volumenteile) chromatographiert. Entfernen des Lösungsmittels aus dem Eluat und Umkristallisation aus Methanol lieferte 4-Ethoxy-2-hydroxy-6-methoxy-N-(p-methoxybenzyl)-thiobenzamid in Form hellgelber Nadeln vom Schmelzpunkt 98—99° C.

## Beispiel 16

In Analogie zu Beispiel 15 wurden unter Verwendung von 1-(4-Ethoxy-2-hydroxy-6-methoxyphenyl)-3-(4-methoxyphenyl)-1-propanon anstelle von 4-Ethoxy-2-hydroxy-6-methoxy-N-(p-methoxybenzyl)benzamid das 1-(4-Ethoxy-2-hydroxy-6-methoxyphenyl)-3-(4-methoxyphenyl)-1-propanthion in Form oranger Nadeln vom Schmelzpunkt 69—70° C (aus Methanol) erhalten.

Beispiel 17

Ein Gemisch von 165 mg 1-(4-Ethoxy-2-hydroxy-6-methoxyphenyl)-3-(4-methoxyphenyl)-1-propanon, 0,2 ml Acetanhydrid und 5 mg Natriumacetat wurde 3 Stunden im verschlossenen Rohr auf 140°C erhitzt. Nach Abkühlen wurde das Gemisch in 20 ml Wasser gegossen, mit 50 ml Chloroform extrahiert und der Extrakt mit Wasser gewaschen, über Natriumsulfat getrocknet und eingedampft. Man erhielt 178 mg eines öligen Rückstandes, der aus Methanol umkristallisiert 138 mg 5-Ethoxy-3-methoxy-2-[3-(4-methoxyphenyl)-propionyl]phenyl-acetat in Form farbloser Nadeln vom Schmelzpunkt 58—59°C lieferte.

Beispiel 18

Zu einer Lösung von 200 mg 4-Ethoxy-2-hydroxy-6-methoxy-N-(p-methoxybenzyl)benzamid in 2 ml Pyridin wurden 0,07 ml Acetanhydrid gegeben. Das Gemisch wurde 18 Stunden bei Raumtemperatur gerührt und dann unter vermindertem Druck zu einem öligen Rückstand eingedampft. Umkristallisation aus Methanol lieferte 60 mg 2[(p-Anisylamino)carbonyl]-5-ethoxy-3-methoxyphenyl-acetat in Form farbloser Nadeln vom Schmelzpunkt 121—122°C.

Beispiel 19

Zu einer Lösung von 317 mg 2-Hydroxy-4,6-dimethoxy-N-(p-methoxybenzyl)benzamid, 0,14 ml Triäthylamin und 24 mg 4-(Dimethylamino)pyridin in 20 ml Dichlormethan wurden 0,1 ml Ethoxycarbonylchlorid gegeben. Das Gemisch wurde 1 Stunde bei Raumtemperatur gerührt, nacheinander mit verdünnter Salzsäure und Wasser gewaschen, über Natriumsulfat getrocknet und eingedampft, wobei 480 mg eines gelben Öls erhalten wurden. Das Öl wurde an Silicagel mit Hexan/Äthylacetat (3 : 1 Volumenteile) chromatographiert. Entfernung des Lösungsmittels aus dem Eluat und Umkristallisation aus Äther/Petroläther lieferte 110 mg 2-[(p-Anisylamino)carbonyl]-3,5-dimethoxyphenylethylcarbonat als farblose Kristalle vom Schmelzpunkt 123—124,5°C.

Beispiel 20

In Analogie zu Beispiel 19 wurde unter Verwendung von 1-(4-Ethoxy-2-hydroxy-6-methoxyphenyl)-3-(4-methoxyphenyl)-1-propanon anstelle von 2-Hydroxy-4,6-dimethoxy-N-(p-methoxybenzyl)benzamid das 5-Ethoxy-3-methoxy-2-[3-(4-methoxyphenyl)propionyl]-phenyl-ethylcarbonat erhalten. $^1$H-NMR-Spektrum (in Chloroform): $\delta$ 1,38 (3H), 1,40 (3H), 3,00 (4H), 3,76 (6H), 4,02 (2H), 4,34 (2H), 6,32 (2H), 6,80 (2H) und 7,13 ppm (2H).

Beispiel 21

Zu einer Lösung von 200 mg 2-Hydroxy-4,6-dimethoxy-N-(p-methoxybenzyl)benzamid in 5 ml Pyridin wurden 347 mg Stearinsäureanhydrid gegeben. Das Gemisch wurde 22,5 Stunden auf 60°C erwärmt, dann unter vermindertem Druck zu einem öligen Rückstand eingedampft, der in 30 ml Äthylacetat gelöst wurde. Die Lösung wurde nacheinander mit verdünnter Salzsäure und Kochsalzlösung gewaschen, über Natriumsulfat getrocknet und eingedampft. Umkristallisation des Rückstandes aus Äthylacetat lieferte 92 mg 2-[(p-Anisylamino)carbonyl]-3,5-dimethoxyphenyl-octadecanoat in Form farbloser Nadeln vom Schmelzpunkt 88—89°C.

Beispiel 22

In Analogie zu Beispiel 21 wurde unter Verwendung von N-[p-(Allyloxy)benzyl-2-hydroxy-4,6-dimethoxybenzamid anstelle von 2-Hydroxy-4,6-dimethoxy-N-(p-methoxybenzyl)-benzamid das 2-{[p-(Allyloxy)benzyl]amino]carbonyl}-3,5-dimethoxyphenyl-octadecanoat in Form farbloser Kristalle vom Schmelzpunkt 88—89°C (aus Äthanol (Hexan) erhalten.

Beispiel 23

In Analogie zu Beispiel 21 wurde unter Verwendung von 1-(4-Ethoxy-2-hydroxy-6-methoxyphenyl)-3-(4-methoxyphenyl)-1-propanon anstelle von 2-Hydroxy-4,6-dimethoxy-N-(p-methoxybenzyl)benzamid das 5-Ethoxy-3-methoxy-2-[3-(4-methoxyphenyl)propionyl]phenyl-octadecanoat vom

Schmelzpunkt 40—41°C (aus Methanol) erhalten.

Beispiel 24

In Analogie zu Beispiel 19 wurde unter Verwendung von Benzoylchlorid anstelle von Ethoxycarbonylchlorid das 2-[(p-Anisylamino)carbonyl]-3,5-dimethoxyphenyl-benzoat in Form farbloser Kristalle vom Schmelzpunkt 124,5—125;5°C (aus Äthylacetat/Hexan) erhalten.

Beispiel 25

In Analogie zu Beispiel 19 wurde unter Verwendung von N-[p-(Allyloxy)benzyl]-2-hydroxy-4,6-dimethoxybenzamid und Benzoylchlorid anstelle von 2-Hydroxy-4,6-dimethoxy-N-(p-methoxybenzyl)benzamid und Ethoxycarbonylchlorid das 2-{[[p-(Allyloxy)benzyl]amino]carbonyl}-3,5-dimethoxyphenylbenzoat in Form farbloser Kristalle vom Schmelzpunkt 105—106°C (aus Methanol) erhalten.

Beispiel 26

In Analogie zu Beispiel 19 wurde unter Verwendung von 1-(4-Ethoxy-2-hydroxy-6-methoxyphenyl)-3-(4-methoxyphenyl)-1-propanon und Benzoylchlorid anstelle von 2-Hydroxy-4,6-dimethoxy-N-(p-methoxybenzyl)benzamid und Ethoxycarbonylchlorid das 5-Ethoxy-3-methoxy-2-[3-(4-methoxyphenyl)propionyl]phenyl-benzoat erhalten. $^1$H-NMR-Spektrum: $\delta$ 1,42 (3H), 2,65, 3,35 (4H), 3,75 (3H), 3,80 (3H), 4,06 (2H), 6,40 (2H), 6,75 (2H), 7,11 (2H) und 7,42—7,70 ppm (3H).

Beispiel 27

Zu einer Lösung von 990 mg 1-(4-Ethoxy-2-hydroxy-6-methoxyphenyl)-3-(4-methoxyphenyl)-1-propanon und 2 ml N,N-Diisopropyläthylamin in 20 ml Toluol wurden auf einmal 10 ml Phosphoroxychlorid gegeben. Das Gemisch wurde 1,5 Stunden bei Raumtemperatur gerührt und dann unter vermindertem Druck bei einer Badtemperatur unterhalb 40°C zu einem öligen Rückstand eingedampft, der in 10 ml Toluol gelöst wurde. Nach Entfernung des Lösungsmittels unter vermindertem Druck wurde der erhaltene ölige Rückstand in 40 ml Tetrahydrofuran/Wasser (1 : 3 Volumenteile) gelöst. Die Lösung wurde 50 Minuten bei Raumtemperatur kräftig gerührt und unter vermindertem Druck bei einer Badtemperatur von 30—40°C zu einer wäßrigen Lösung konzentriert, die dann dreimal mit 50 ml Chloroform extrahiert wurde. Die vereinigten Chloroformextrakte wurden mit wenig Wasser gewaschen, über Natriumsulfat getrocknet und zu einem öligen Rückstand eingedampft, der in 100 ml 0,1N Kaliumcarbonat gelöst wurde. Die Lösung wurde zweimal mit 30 ml Äthylacetat gewaschen, mit Salzsäure angesäuert und dreimal mit je 50 ml Äther extrahiert. Die vereinigten Ätherextrakte wurden mit wenig Wasser gewaschen, über Natriumsulfat getrocknet und zu einem öligen Rückstand eingedampft. Umkristallisation aus Äther lieferte 930 mg 5-Ethoxy-3-methoxy-2-[3-(4-methoxyphenyl)propionyl]phenyldihydrogenphosphat in Form farbloser Nadeln vom Schmelzpunkt 122—125°C (Zersetzung].

Beispiel 28

410 mg 5-Ethoxy-3-methoxy-2-[3-(4-methoxyphenyl)-propionyl]phenyldihydrogenphosphat wurden in 15 ml 0,1N Natronlauge gelöst. Nach sorgfältigem Einstellen des pHs auf 8,0 mit 0,1N Natronlauge wurde die Lösung zu einem weißen Feststoff lyophilisiert. Umkristallisation des Feststoffes aus Wasser/Acetonitril lieferte 380 mg Dinatrium 5-ethoxy-3-methoxy-2-[3-(4-methoxyphenyl)-propionyl]phenylphosphat in Form farbloser Nadeln vom Schmelzpunkt 138—139°C.

Beispiel 29

Zu einer gerührten Lösung von 380 mg Dibenzylphosphorochloridat in 10 ml Benzol wurde eine Lösung gegeben, die 350 mg 4-Ethoxy-2-hydroxy-6-methoxy-N-(p-methoxybenzyl)-benzamid und 51 mg 60%iges Natriumhydrid in 10 ml Dimethylformamid enthielt. Nach 14,5 Stunden Rühren bei Raumtemperatur wurde das Gemisch mit 50 ml Äthylacetat verdünnt, dreimal mit je 50 ml Wasser gewaschen, über Natriumsulfat getrocknet und eingedampft. Man erhielt 1,25 g gelbes Öl, das an 37,5 g Silicagel mit Äthylacetat/Hexan (1 : 1 Volumenteile) eluiert wurde. Entfernung des Lösungsmittels lieferte 237 mg Dibenzyl 2-[(p-Anisylamino)carbonyl]-5-ethoxy-3-methoxyphenylphosphat in Form

eines farblosen Sirups, der in 20 ml Chloroform gelöst wurde.

Die so erhaltene Lösung wurde 34 Stunden bei Raumtemperatur und Atmosphärendruck in Gegenwart von 47 mg 10%igen Palladium/Kohle-Katalysator hydriert. Entfernung des Katalysators und Eindampfen des Filtrates lieferte 94 mg eines weißen Rückstandes, der aus Methanol umkristallisiert 79 mg 2-[(p-Anisylamino)carbonyl]-5-ethoxy-3-methoxyphenyl-dihydrogenphosphat in Form farbloser Kristalle vom Schmelzpunkt 162—163,5°C lieferte. Die so erhaltene Verbindung wurde in das Dinatriumsalz vom Schmelzpunkt 126—127°C in Analogie zu dem Verfahren von Beispiel 28 übergeführt.

## Beispiel 30

In Analogie zu Beispiel 29 wurde mit 2-Hydroxy-4,6-dimethoxy-N-(p-methoxybenzyl)benzamid anstelle von 4-Ethoxy-2-hydroxy-6-methoxy-N-(p-methoxybenzyl)benzamid, das 2-[(p-Anisylamino)carbonyl]-3,5-dimethoxyphenyldihydrogenphosphat in Form farbloser Kristalle vom Schmelzpunkt 160,5—162,5°C erhalten.

## Beispiel 31

Zu einer gerührten Lösung von 300 mg 2-Hydroxy-4,6-dimethoxy-N-(p-methoxybenzyl)benzamid und 50 mg 60%igen Natriumhydrid in 3 ml Dimethylformamid wurden 600 mg 2,3,4,6-Tetra-O-acetyl-α-D-glucopyranosylbromid gegeben. Das Gemisch wurde 18 Stunden bei Raumtemperatur gerührt und dann mit 50 ml Äthylacetat und 30 ml Wasser versetzt. Die Äthylacetatphase wurde abgetrennt, mit Wasser gewaschen, über Natriumsulfat getrocknet und zu einem öligen Rückstand eingedampft. Der Rückstand wurde an 10 g Silicagel mit Äthylacetat chromatographiert. Entfernung des Lösungsmittels aus dem Eluat und Umkristallisation aus Methanol lieferte 135 mg 2-[(p-Anisylamino)carbonyl]-3,5-dimethoxyphenyl-tetra-O-acetyl-β-D-glucopyranosid in Form farbloser Kristalle vom Schmelzpunkt 73:76°C.

## Beispiel 32

Zu einer Suspension von 100 mg 2-[(p-Anisylamino)-carbonyl]-3,5-dimethoxyphenyl-tetra-O-acetyl-β-D-glucopyranosid in 30 ml Methanol wurden 0,09 ml Wasser und 0,09 ml Triäthylamin gegeben. Nach 3 Tagen Rühren bei Raumtemperatur wurde das Gemisch zu einem festen Rückstand eingedampft. Der Rückstand wurde an 10 g Silicagel mit Äthylacetat/Methanol (10 : 1 Volumenteile) chromatographiert. Entfernung des Lösungsmittels und Umkristallisation aus Äthanol/Hexan lieferte 40 mg 2-[(p-Anisylamino)carbonyl]-3,5-dimethoxyphenyl-β-D-glucopyranosid in Form farbloser Nadeln vom Schmelzpunkt 132—133°C.

## Beispiel 33

Zu einer gerührten Lösung von 330 mg 1-(4-Ethoxy-2-hydroxy-6-methoxyphenyl)-3-(4-methoxyphenyl)-1-propanon und 44 mg 60%iges Natriumhydrid in 5 ml Dimethylformamid wurde Tetra-O-acetyl-α-D-glucopyranosylbromid gegeben. Das Gemisch wurde 18 Stunden bei Raumtemperatur gerührt, mit 30 ml kaltem Wasser verdünnt und mit dreimal 30 ml Äthylacetat extrahiert. Die vereinigten Extrakte wurden mit Wasser gewaschen, über Natriumsulfat getrocknet und eingedampft. Man erhielt 700 mg rohes 5-Ethoxy-3-methoxy-2-[3-(4-methoxyphenyl)propionyl]phenyl-tetra-O-acetyl-β-D-glucopyranosid.

Das vorstehende Rohmaterial wurde durch Chromatographie an Silicagel mit Hexan/Äthylacetat (3 : 1 Volumenteile) gereinigt und dann gemäß dem Verfahren von Beispiel 32 verseift, wobei 5-Ethoxy-3-methoxy-2-[3-(4-methoxyphenyl)propionyl]phenyl-β-D-glucopyranosid erhalten wurde. $^1$H NMR: δ 1,35 (3H), 2,6—3,2 (4H), 3,2—4,6 (6H), 3,72 (H), 3,78 (3H), 4,05 (2H), 4,87 (1H), 6,26 (1H), 6,45 (1H), 6,78 (2H) und 7,15 ppm (2H).

## Beispiel 34

In Analogie zu Beispiel 12 wurde mit 2'-Hydroxy-6'-methoxy-4'-propoxyacetophenon anstelle von 4'-Ethoxy-2'-hydroxy-6'-methoxyacetophenon das 2-Hydroxy-6-methoxy-4-propoxy-N-(p-methoxybenzyl)benzamid in Form farbloser Kristalle vom Schmelzpunkt 96,5—97,5°C (aus Methanol) erhalten.

Beispiel 35

In Analogie zu Beispiel 12 wurde mit 2'-Hydroxy-6'-methoxy-4'-(2-propenyloxy)acetophenon anstelle von 4'-Ethoxy-2'-hydroxy-6'-methoxyacetophenon das 4-(Allyloxy)-2-hydroxy-6-methoxy-N-(p-methoxybenzyl)benzamid in Form farbloser Kristalle vom Schmelzpunkt 69,5—70° C (aus Methanol) erhalten.

Beispiel 36

In Analogie zu Beispiel 12 wurde mit 2'-Hydroxy-6'-methoxy-4'-(3-methyl-2-butenyloxy)acetophenon anstelle von 4'-Ethoxy-2'-hydroxy-6'-methoxyacetophenon das 2-Hydroxy-6-methoxy-4-(3-methyl-2-butenyloxy)-N-(p-methoxybenzyl)benzamid in Form farbloser Kristalle vom Schmelzpunkt 48—48,5° C (aus Petroläther) erhalten.

Beispiel 37

In Analogie zu Beispiel 29 wurde mit 2-Hydroxy-6-methoxy-4-propoxy-N-(p-methoxybenzyl)benzamid anstelle von 4-Ethoxy-2-hydroxy-6-methoxy-N-(p-methoxybenzyl)-benzamid das Dinatrium 2-[(p-anisylamino)carbonyl]-3-methoxy-5-propoxyphenylphosphat in Form farbloser Kristalle vom Schmelzpunkt 116—119° C (aus Äthylacetat) erhalten.

Beispiel A

Tabletten mit folgenden Inhaltsstoffen können nach üblichen Verfahren hergestellt werden:

| | |
|---|---|
| Aktiver Inhaltsstoff (Verbindung der Formel I) | 300 mg |
| Trockene Lactose | 200 mg |
| Mikrokristalline Cellulose | 30 mg |
| Polyvinylpyrrolidon | 5 mg |
| Magnesiumnstearat | 4 mg |

Beispiel B

Tropfen für intranasale Verabreichung können nach an sich bekannten Verfahren mit folgenden Inhaltsstoffen hergestellt werden:

| | |
|---|---|
| Aktiver Inhaltsstoff (Verbindung der Formel I) | 0,1 mg |
| Oberflächenaktives Mittel | 0,05 mg |
| Propylenglykol/Wasser (1 : 1 Volumenteile) ad | 1 ml |

Ein annehmbarer Konzentrationsbereich für den aktiven Wirkstoff ist 0,001 bis 1 mg/ml.

Beispiel C

Pastillen mit den folgenden Inhaltsstoffen können in an sich bekannter Weise hergestellt werden:

| | |
|---|---|
| Aktiver Inhaltsstoff (Verbindung der Formel I) | 0,1 g |
| Puderzucker | 1,6 g |
| Akaziengummi | 0,2 g |
| Dextrin | 0,1 g |
| Geschmacksstoffe | 0,001 g |

**Patentansprüche für die Vertragsstaaten: BE, CH, DE, FR, GB, IT, LI, LU, NL, SE**

1. Verbindungen der Formel

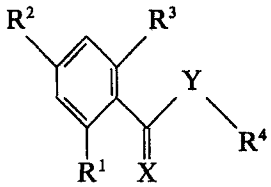

(I)

worin X Sauerstoff, Schwefel oder Hydroxyimino; Y Methylen oder Imino; $R^1$ Hydroxy, Phosphonooxy, Glycosyloxy, acyliertes Glycosyloxy, Acyloxy oder nieder-Alkoxycarbonyloxy; $R^2$ nieder-Alkoxy, nieder-Alkenyloxy oder nieder-Alkylthio; R3 nieder-Alkoxy; $R^4$ nieder-Alkyl, p-nieder-Alkoxybenzoyl oder substituiertes oder unsubstituiertes Phenyl, Benzyl, Pyridylmethyl, Furfuryl, Tetrahydrofurfuryl, Thienyl, Tetrahydrothenyl, Pyrrolylmethyl, Pyrrolinylmethyl oder Pyrrolidinylmethyl darstellt, mit der Bedingung, daß Y nicht Methylen ist, wenn $R^4$ Alkyl, Phenyl, substituiertes Phenyl, Benzyl oder p-Hydroxybenzyl ist und mit der weiteren Bedingung, daß $R^2$ und $R^3$ voneinander verschieden sind, wenn $R^4$ p-Methoxybenzyl und Y Methylen ist,
und pharmazeutisch anwendbare Salze davon.

2. Verbindungen gemäß Anspruch 1, in denen X Sauerstoff, Schwefel oder Hydroxyimino; Y Methylen oder Imino; $R^1$ Hydroxy, Phosphonooxy, Glycosyloxy, acyliertes Glycosyloxy, Acyloxy oder nieder-Alkoxycarbonyloxy; $R^2$ nieder-Alkoxy oder nieder-Alkylthio; $R^3$ nieder-Alkoxy; $R^4$ nieder-Alkyl, p-nieder-Alkoxybenzoyl oder substituiertes oder unsubstituiertes Phenyl, Benzyl, Pyridylmethyl, Furfuryl, Tetrahydrofurfuryl, Thenyl, Tetrahydrothenyl, Pyrrolylmethyl, Pyrrolinylmethyl oder Pyrrolidinylmethyl ist; mit der Bedingung, daß nicht Methylen ist, wenn $R^4$ Alkyl, Phenyl, substituiertes Phenyl, Benzyl oder p-Hydroxybenzyl ist, und mit der weiteren Bedingung, daß $R^2$ und $R^3$ voneinander verschieden sind, wenn $R^4$ p-Methoxybenzyl und Y Methylen ist.

3. Verbindungen gemäß Anspruch 1, in denen $R^1$ Hydroxy, nieder-Alkanoyloxy, Benzoyloxy, Phosphonooxy, nieder-Alkoxycarbonyloxy; $R^2$ nieder-Alkoxy oder nieder-Alkenyloxy; $R^3$ nieder-Alkoxy; $R^4$ substituiertes Phenyl; X Sauerstoff oder Schwefel und Y Methylen oder Imino ist.

4. Verbindungen gemäß Anspruch 3, in denen Y Imino ist.

5. 4-Ethoxy-2-hydroxy--6-methoxy-N-(p-methoxybenzyl)-benzamid.

6. 2-[(p-Anisylamino)carbonyl]-5-ethoxy-3-methoxyphenyl-dihydrogenphosphat und dessen Dinatriumsalz.

7. 1-(4-Ethoxy-2-hydroxy-6-methoxyphenyl)-3-(4-methoxyphenyl)-1-propanon.

8. 5-Ethoxy-3-methoxy-2-[3-(p-methoxyphenyl)-propionyl]phenyl-acetat.

9. 2-Hydroxy-4,6-dimethoxy-N-[p-(methylthio)benzyl]-benzamid.

10. 2.{[[p-(Allyloxy)benzyl]amino]carbonyl}-3,5-dimethoxyphenyl-benzoat.

11. 1-(4-Ethoxy-2-hydroxy-6-methoxyphenyl)-3-(4-methoxyphenyl)-1-propanthion.

12. 5-Ethoxy-3-methoxy-2-[3-(4-methoxyphenyl)-propionyl]phenyl-ethylcarbonat.

13. 2-Hydroxy-6-methoxy-4-propoxy-N-(p-methoxybenzyl)-benzamid.

14. 4-(Allyloxy)-2-hydroxy-6-methoxy-N-(p-methoxybenzyl)benzamid.

15. 2-Hydroxy-6-methoxy-4-(3-methyl-2-butenyloxy)-N-(p-methoxybenzyl)benzamid.

16. Dinatrium 2-[p-anisylamino)-carbonyl]-3-methoxy-5-propoxyphenyl-phosphat.

17. 4-Ethoxy-2-hydroxy-6-methoxy-N-(p-methoxybenzyl)benzamid.

18. Verbindungen der Formel I als Mittel gegen Viruserkrankungen.

19. Verfahren zur Herstellung von Verbindungen der Formel I, dadurch gekennzeichnet, daß man

a) eine Verbindung der Formel

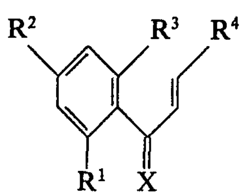

(II)

worin X, $R^1$, $R^2$, $R^3$ und $R^4$ wie in Formel I sind,
in Gegenwart eines Katalysators in einem Lösungsmittel hydriert, oder

b) ein reaktionsfähiges Derivat einer Carbonsäure der Formel

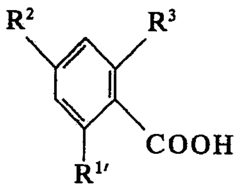

(III)

worin $R^2$ und $R^3$ wie in Formel I sind und $R^{1'}$ eine geschützte Hydroxygruppe ist, mit einer Verbindung der Formel

$R^4-NH_2$ (IV)

worin $R^4$ wie in Formel I ist, umsetzt und sodann die Schutzgruppe von der Hydroxygruppe entfernt, oder

c) ein Acetophenon der Formel

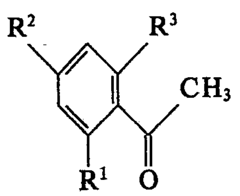

(V)

worin $R^1$, $R^2$ und $R^3$ wie in Formel I sind, mit Jod in einem tertiären Amin umsetzt und das erhaltene Salze mit einem Amin der obigen Formel IV umsetzt, oder

d) einen Ester der Formel

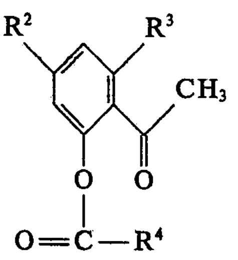

(VI)

worin $R^2$, $R^3$ und $R^4$ wie in Formel I sind, in Gegenwart einer Base in einem Lösungsmittel umlagert, oder

e) ein Keton der Formel

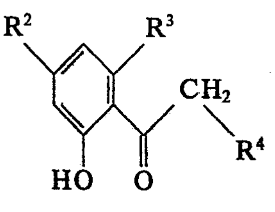

(VII)

worin $R^2$, $R^3$ und $R^4$ wie in Formel I sind, mit einem Hydroxylaminsalz in einem Lösungsmittel umsetzt, oder

f) eine Carbonylverbindung der Formel

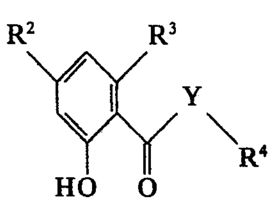

(VIII)

worin Y, $R^2$, $R^3$ und $R^4$ wie in Form I sind,
mit Phosphorpentasulfid in Gegenwart einer Base in einem Lösungsmittel umsetzt, oder

g) ein Phenol der Formel

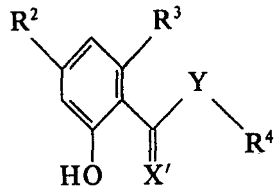

(IX)

worin Y, $R^2$, $R^3$ und $R^4$ wie in Formel I sind und X' Sauerstoff oder Schwefel ist,
mit einem Acylierungsmittel in Gegenwart einer Base umsetzt, oder

h) ein Phenol der obigen Formel IX mit Phosphoroxychlorid oder Dibenzylphosphorochloridat in Gegenwart einer Base in einem Lösungsmittel umsetzt und das Reaktionsprodukt hydrolysiert bzw. hydrogenolysiert und gewünschtenfalls eine erhaltene Verbindung in ein Salz überführt, oder

i) ein Phenol der obigen Formel IX mit einem Glycosylhalogenid oder einem acylierten Glycosylhalogenid in Gegenwart eines Katalysators in einem Lösungsmittel umsetzt und gewünschtenfalls danach die Acylgruppen entfernt.

20. Pharmazeutische Präparate, gekennzeichnet durch einen Gehalt an einer Verbindung der Formel I.

**Patentansprüche für den Vertragsstaat: AT**

1. Verfahren zur Herstellung von Verbindungen der Formel

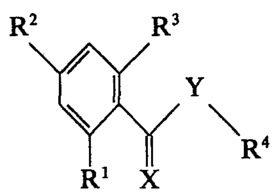

(I)

worin X Sauerstoff, Schwefel oder Hydroxyimini; Y Methylen oder Imino; $R^1$ Hydroxy, Phosphonooxy, Glycosyloxy, acyliertes Glycosyloxy, Acyloxy oder nieder-Alkoxycarbonyloxy; $R^2$ nieder-Alkoxy, nieder-Alkenyloxy oder nieder-Alkylthio; $R^3$ nieder-Alkoxy; $R^4$ nieder-Alkyl, p-nieder-Alkoxybenzoyl oder substituiertes oder unsubstituiertes Phenyl, Benzyl, Pyridylmethyl, Furfuryl, Tetrahydrofurfuryl, Thienyl, Tetrahydrothenyl, Pyrrolylmethyl, Pyrrolinylmethyl oder Pyrrolidinylmethyl darstellt, mit der Bedingung, daß Y nicht Methylen ist, wenn $R^4$ Alkyl, Phenyl, substituiertes Phenyl, Benzyl oder p-Hydroxybenzyl ist und mit der weiteren Bedingung, daß $R^2$ und $R^3$ voneinander verschieden sind, wenn $R^4$ p-Methoxybenzyl und Y Methylen ist,
und pharmazeutisch anwendbare Salze davon, dadurch gekennzeichnet, daß man

a) eine Verbindung der Formel

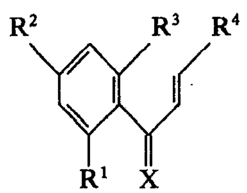

(II)

worin X, $R^1$, $R^2$, $R^3$ und $R^4$ wie in Formel I sind,
in Gegenwart eines Katalysators in einem Lösungsmittel hydriert, oder

b) ein reaktionsfähiges Derivat einer Carbonsäure der Formel

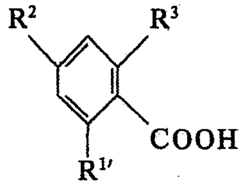

(III)

worin $R^2$ und $R^3$ wie in Formel I sind und $R^{1'}$ eine geschützte Hydroxygruppe ist, mit einer Verbindung der Formel

$R^4—NH_2$ (IV)

worin $R^4$ wie in Formel I ist, umsetzt und sodann die Schutzgruppe von der Hydroxygruppe entfernt, oder

c) ein Acetophenon der Formel

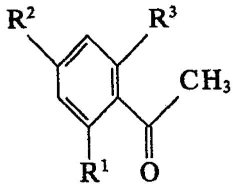

(V)

worin $R^1$, $R^2$ und $R^3$ wie in Formel I sind, mit Jod in einem tertiären Amin umsetzt und das erhaltene Salze mit einem Amin der obigen Formel IV umsetzt, oder

d) einen Ester der Formel

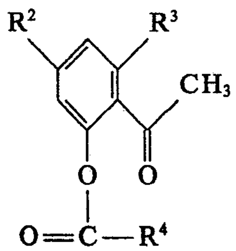

(VI)

worin $R^2$, $R^3$ und $R^4$ wie in Formel I sind, in Gegenwart einer Base in einem Lösungsmittel umlagert, oder

e) ein Keton der Formel

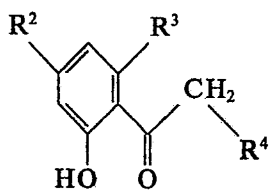

(VII)

worin $R^2$, $R^3$ und $R^4$ wie in Formel I sind, mit einem Hydroxylaminsalz in einem Lösungsmittel umsetzt, oder

f) eine Carbonylverbindung der Formel

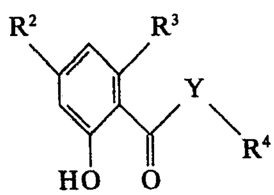

(VIII)

worin Y, $R^2$, $R^3$ und $R^4$ wie in Formel I sind,
mit Phosphorpentasulfid in Gegenwart einer Base in einem Lösungsmittel umsetzt, oder

g) ein Phenol der Formel

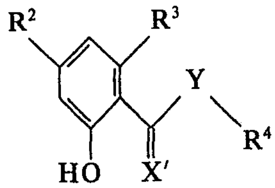

(IX)

worin Y, $R^2$, $R^3$ und $R^4$ wie in Formel I sind und X' Sauerstoff oder Schwefel ist,
mit einem Acylierungsmittel in Gegenwart einer Base umsetzt, oder

h) ein Phenol der obigen Formel IX mit Phosphoroxychlorid oder Dibenzylphosphorochloridat in Gegenwart einer Base in einem Lösungsmittel umsetzt und das Reaktionsprodukt hydrolysiert bzw. hydrogenolysiert und gewünschtenfalls eine erhaltene Verbindung in ein Salz überführt, oder

i) ein Phenol der obigen Formel IX mit einem Glycosylhalogenid oder einem acylierten Glycosylhalogenid in Gegenwart eines Katalysators in einem Lösungsmittel umsetzt und gewünschtenfalls danach die Acylgruppen entfernt.

2. Verfahren nach Anspruch 1, dadurch gekennzeichnet, daß man Verbindungen gemäß Anspruch 1, in denen X Sauerstoff, Schwefel oder Hydroxyimino; Y Methylen oder Imino; $R^1$ Hydroxy, Phosphonooxy, Glycosyloxy, acyliertes Glycosyloxy, Acyloxy oder nieder-Alkoxycarbonyloxy; $R^2$ nieder-Alkoxy oder nieder-Alkylthio; $R^3$ nieder-Alkoxy; $R^4$ nieder-Alkyl, p-nieder-Alkoxybenzoyl oder substituiertes oder unsubstituiertes Phenyl, Benzyl, Pyridylmethyl, Furfuryl, Tetrahydrofuryl, Thienyl, Tetrahydrothenyl, Pyrrolylmethyl, Pyrrolinylmethyl oder Pyrrolidinylmethyl mit der Bedingung, daß Y nicht Methylen ist, wenn $R^4$ Alkyl, Phenyl, substituiertes Phenyl, Benzyl oder p-Hydroxybenzyl ist, und mit der weiteren Bedingung, daß $R^2$ und $R^3$ voneinander verschieden sind, wenn $R^4$ p-Methoxybenzyl und Y Methylen ist, herstellt.

3. Verfahren nach Anspruch 1, dadurch gekennzeichnet, daß man Verbindungen gemäß Anspruch 1, in denen $R^1$ Hydroxy, nieder-Alkanoyloxy, Benzoyloxy, Phosphonooxy, nieder-Alkoxycarbonyloxy; $R^2$ nieder-Alkoxy oder nieder-Alkenyloxy; $R^3$ nieder-Alkoxy; $R^4$ substituiertes Phenyl; X Sauerstoff oder Schwefel und Y Methylen oder Imino ist, herstellt.

4. Verfahren nach Anspruch 3, dadurch gekennzeichnet, daß man Verbindungen der Formel I, in denen Y Imino ist, herstellt.

5. Verfahren nach Anspruch 1, dadurch gekennzeichnet, daß man 4-Ethoxy-2-hydroxy-6-methoxy-N-(p-methoxybenzyl)-benzamid herstellt.

6. Verfahren nach Anspruch 1, dadurch gekennzeichnet,daß man 2-[(p-Anisylamino)carbonyl]-5-ethoxy-3-methoxyphenyl-dihydrogenphosphat und dessen Dinatriumsalz herstellt.

7. Verfahren nach Anspruch 1, dadurch gekennzeichnet, daß man 1-(4-Ethoxy-2-hydroxy-6-methoxyphenyl)-3-(4-methoxyphenyl)-1-propanon herstellt.

8. Verfahren nach Anspruch 1, dadurch gekennzeichnet, daß man 5-Ethoxy-3-methoxy-2-[3-(p-methoxyphenyl)-propionyl]phenyl-acetat herstellt.

9. Verfahren nach Anspruch 1, dadurch gekennzeichnet, daß man 2-Hydroxy-4,6-dimethoxy-N-[p-(methylthio)benzyl]-benzamid herstellt.

10. Verfahren nach Anspruch 1, dadurch gekennzeichnet, daß man 2-{[[p-(Allyloxy)benzyl]amino]carbonyl}-3,5-dimethoxyphenyl-benzoat herstellt.

11. Verfahren nach Anspruch 1, dadurch gekennzeichnet, daß man 1-(4-Ethoxy-2-hydroxy-6-methoxyphenyl)-3-(4-methoxyphenyl)-1-proanthion herstellt.

12. Verfahren nach Anspruch 1, dadurch gekennzeichnet, daß man 5-Ethoxy-3-methoxy-2-[3-(4-methoxyphenyl)-propionyl]phenyl-ethylcarbonat herstellt.

13. Verfahren nach Anspruch 1, dadurch gekennzeichnet, daß man 2-Hydroxy-6-methoxy-4-propoxy-N-(p-methoxybenzyl)-benzamid herstellt.

14. Verfahren nach Anspruch 1, dadurch gekennzeichnet, daß man 4-(Allyloxy)-2-hydroxy-6-methoxy-N-(p-methoxybenzyl)benzamid herstellt.

15. Verfahren nach Anspruch 1, dadurch gekennzeichnet, daß man 2-Hydroxy-6-methoxy-4-(3-methyl-2-butenyloxy)-N-(p-methoxybenzyl)benzamid herstellt.

16. Verfahren nach Anspruch 1, dadurch gekennzeichnet, daß man Dinatrium 2-[p-anisylamino)-carbonyl]-3-methoxy-5-propoxyphenyl-phosphat herstellt.

**Claims for the Contracting States: BE, CH, DE, FR, GB, IT, LI, LU, NL, SE**

1. Compounds of the formula

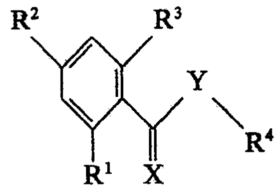

(I)

wherein X represents oxygen, sulphur or hydroxyimino; Y represents methylene or imino; $R^1$ represents hydroxy, phosphonooxy, glycosyloxy, acylated glycosyloxy, acyloxy or lower-alkoxy-carbonyloxy; $R^2$ represents lower-alkoxy, lower-alkenyloxy or lower-alkylthio; $R^3$ represents lower-alkoxy; $R^4$ represents lower-alkyl, p-lower-alkoxybenzoyl or substituted or unsubstituted phenyl, benzyl, pyridylmethyl, furfuryl, tetrahydrofurfuryl, thienyl, tetrahydrothenyl, pyrrolylmethyl, pyrrolinylmethyl or pyrrolidinylmethyl, with the proviso that Y is not methylene when $R^4$ is alkyl, phenyl, substituted phenyl, benzyl or p-hydroxy-benzyl and with the further proviso that $R^2$ and $R^3$ are different from one another when $R^4$ is p-methoxybenzyl and Y is methylene,

and pharmaceutically usable salts thereof.

2. Compounds in accordance with claim 1, in which X is oxygen, sulphur or hydroxyimino; Y is methylene or imino; $R^1$ is hydroxy, phosphonooxy, glycosyloxy, acylated glycosyloxy, acyloxy or lower-alkoxycarbonyloxy; $R^2$ is lower-alkoxy or lower-alkylthio; $R^3$ is lower-alkoxy; $R^4$ is lower-alkyl, p-lower-alkoxybenzoyl or substituted or unsubstituted phenyl, benzyl, pyridylmethyl, furfuryl, tetrahydrofurfuryl, thenyl, tetrahydrothenyl, pyrrolylmethyl, pyrrolinylmethyl or pyrrolidinylmethyl; with the proviso that Y is not nethylene when $R^4$ is alkyl, phenyl, substituted phenyl, benzyl or p-hydroxybenzyl and with the further proviso that $R^2$ and $R^3$ are different from one another when $R^4$ is p-methoxybenzyl and Y is methylene.

3. Compounds in accordance with claim 1, in which $R^1$ is hydroxy, lower-alkanoyloxy, benzoyloxy, phosphonooxy, lower-alkoxycarbonyloxy; $R^2$ is lower-alkoxy or lower-alkenyloxy; $R^3$ is lower-alkoxy; $R^4$ is substituted phenyl; X is oxygen or sulphur and Y is methylene or imino.

4. Compounds in accordance with claim 3, in which Y is imino.

5. 4-Ethoxy-2-hydroxy-6-methoxy-N-(p-methoxybenzyl)-benzamide.

6. 2-[(p-Anisylamino)carbonyl]-5-ethoxy-3-methoxyphenyl dihydrogen phosphate and its disodium salt.

7. 1-(4-Ethoxy-2-hydroxy-6-methoxyphenyl)-3-(4-methoxyphenyl)-1-propanone.

8. 5-Ethoxy-3-methoxy-2-[3-(p-methoxyphenyl)-propionyl]phenyl acetate.

9. 2-Hydroxy-4,6-dimethoxy-N-[p-(methylthio)benzyl]-benzamide.

10. 2-{[[p-(Allyloxy)benzyl]amino]carbonyl}-3,5-dimethoxyphenyl benzoate.

11. 1-(4-Ethoxy-2-hydroxy-6-methoxyphenyl)-3-(4-methoxyphenyl)-1-propanethione.

12. 5-Ethoxy-3-methoxy-2-[3-(4-methoxyphenyl)-propionyl]phenyl ethylcarbonate.

13. 2-Hydroxy-6-methoxy-4-propoxy-N-(p-methoxybenzyl)-benzamide.

14. 4-(Allyloxy)-2-hydroxy-6-methoxy-N-(p-methoxybenzyl)benzamide.

15. 2-Hydroxy-6-methoxy-4-(3-methyl-2-butenyloxy)-N-(p-methoxybenzyl)benzamide.

16. Disodium 2-[(p-anisylamino)-carbonyl]-3-methoxy-5-propoxyphenyl phosphate.

17. 4-Ethoxy-2-hydroxy-6-methoxy-N-(p-methoxybenzyl)-benzamide.

18. Compounds of formula I as agents against viral diseases.

19. A process for the manufacture of compounds of formula I, characterized by

a) hydrogenating a compound of the formula

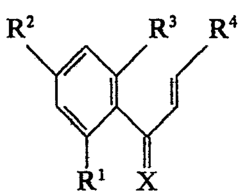

(II)

wherein X, $R^1$, $R^2$, $R^3$ and $R^4$ are as in formula I,

in the presence of a catalyst in a solvent, or

b) reacting a reactive derivative of a carboxylic acid of the formula

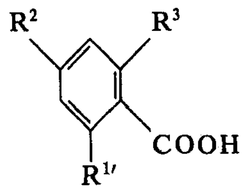

(III)

wherein $R^2$ and $R^3$ are as in formula I and $R^{1'}$ is a protected hydroxy group, with a compound of the formula

$R^4-NH_2$ (IV)

wherein $R^4$ is as in formula I, and then removing the protecting group of the hydroxy group, or

c) reacting an acetophenone of the formula

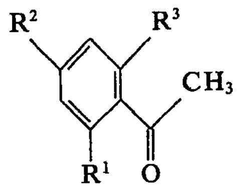

(V)

wherein $R^1$, $R^2$ and $R^3$ are as in formula I, with iodine in a tertiary amine and reacting the salt obtained with an amine of formula IV above, or

d) rearranging an ester of the formula

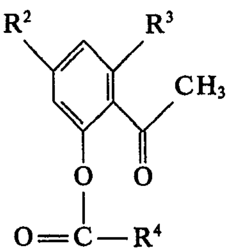

(VI)

wherein $R^2$, $R^3$ and $R^4$ are as in formula I, in the presence of a base in a solvent, or

e) reacting a ketone of the formula

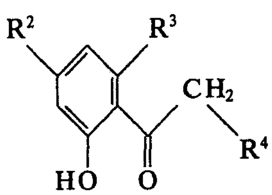

(VII)

wherein $R^2$, $R^3$ and $R^4$ are as in formula I, with a hydroxylamine salt in a solvent, or

f) reacting a carbonyl compound of the formula

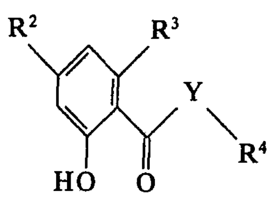

(VIII)

wherein Y, $R^2$, $R^3$ and $R^4$ are as in formula I,

with phosphorus pentasulphide in the presence of a base in a solvent, or

g) reacting a phenol of the formula

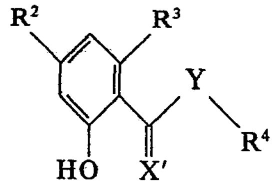

(IX)

wherein Y, $R^2$, $R^3$ and $R^4$ are as in formula I and X' is oxygen or sulphur, with an acylating agent in the presence of a base, or

h) reacting a phenol of formula IX above with phosphorus oxychloride or dibenzyl phosphorochloridate in the presence of a base in a solvent and hydrolyzing or hydrogenolyzing the reaction prodcut and, if desired, converting a compound obtained into a salt, or

i) reacting a phenol of formula IX above with a glycosyl halide or an acylated glycosyl halide in the presence of a catalyst in a solvent and, if desired, thereafter removing the acyl groups,

20. Pharmaceutical preparations, characterized by a content of a compound of formular I.

**Claims for the Contracting State: AT**

1. A process for the manufacture of compounds of the formula

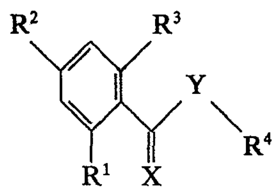

(I)

wherein X represents oxygen, sulphur or hydroxyimino; Y represents methylene or imino; $R^1$ represents hydroxy, phosphonooxy, glycosyloxy, acylated glycosyloxy, acyloxy or lower-alkoxycarbonyloxy; $R^2$ represents lower-alkoxy, lower-alkenyloxy or lower-alkylthio; $R^3$ represents lower-alkoxy; $R^4$ represents lower alkyl, p-lower-alkoxybenzoyl or substituted or unsubstituted phenyl, benzyl, pyridylmethyl, furfuryl, tetrahydrofurfuryl, thienyl, tetrahydrothenyl, pyrrolylmethyl, pyrrolinylmethyl or pyrrolidinylmethyl, with the proviso that Y is not methylene when $R^4$ is alkyl, phenyl, substituted phenyl, benzyl or p-hydroxybenzyl and with the further proviso that $R^2$ and $R^3$ are different from one another when $R^4$ is p-methoxybenzyl and Y is methylene,
and pharmaceutically usable salts thereof, characterized by

a) hydrogenating a compound of the formula

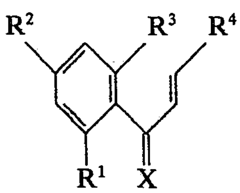

(II)

wherin X, $R^1$, $R^2$, $R^3$ and $R^4$ are as in formula I,
in the presence of a catalyst in a solvent, or

b) reacting a reactive derivative of a carboxylic acid of the formula

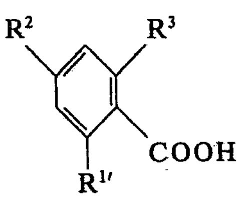

(III)

wherein $R^2$ and $R^3$ are as in formula I and $R^{1'}$ is a protected hydroxy group, with a compound of the formula

$R^4-NH_2$ (IV)

wherein $R^4$ is as in formula I, and then removing the protecting group of the hydroxy group, or

c) reacting an acetophenone of the formula

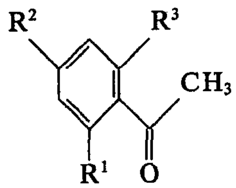

(V)

wherein $R^1$, $R^2$ and $R^3$ are as in formula I, with iodine in a tertiary amine and reacting the salt obtained with an amine of formula IV above, or

d) rearranging an ester of the formula

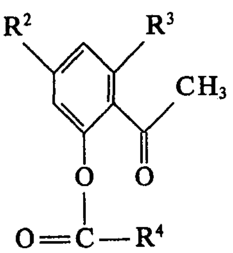

(VI)

wherein $R^2$, $R^3$ and $R^4$ are as in formula I, in the presence of a base in a solvent, or

e) reacting a ketone of the formula

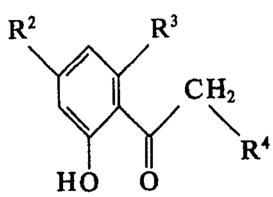

(VII)

wherein $R^2$, $R^3$ and $R^4$ are as in formula I, with a hydroxylamine salt in a solvent, or

f) reacting a carbonyl compound of the formula

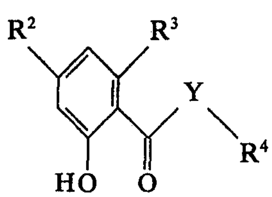

(VIII)

wherein Y, $R^2$, $R^3$ and $R^4$ are as in formula I, with phosphorus pentasulphide in the presence of a base in a solvent, or

g) reacting a phenol of the formula

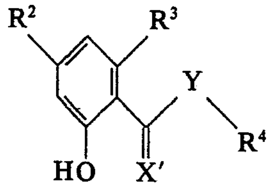

(IX)

wherin Y, $R^2$, $R^3$ and $R^4$ are as in formula I and X′ is oxygen or sulphur, with an acylating agent in the presence of a base, or

h) reacting a phenol of formula IX above with phosphorus oxychloride or dibenzyl phosphorochloridate in the presence of a base in a solvent and hydrolyzing or hydrogenolyzing the reaction product and, if desired, converting a compound obtained into a salt, or

i) reacting a phenol of formula IX above with a glycosyl halide or an acylated glycosyl halide in the presence of a catalyst in a solvent and, if desired, thereafter removing the acyl groups.

2. A process according to claim 1, characterized in that compounds in accordance with claim 1 in which X is oxygen, sulphur or hydroxyimino; Y is methylene or imino; $R^1$ is hydroxy, phosphonooxy, glycosyloxy, acylated glycosyloxy, acyloxy or lower-alkoxycarbonyloxy; $R^2$ is lower-alkoxy or lower-alkylthio; $R^3$ is lower alkoxy; $R^4$ is lower-alkyl, p-lower-alkoxybenzoyl or substituted or unsubstituted phenyl, benzyl, pyridylmethyl, furfuryl, tetrahydrofurfuryl, thienyl, tetrahydrothenyl, pyrrolylmethyl, pyrrolinylmethyl or pyrrolidinylmethyl, with the proviso that Y is not methylene when $R^4$ is alkyl, phenyl, substituted phenyl, benzyl, or p-hydroxybenzyl and with the further proviso that $R^2$ and $R^3$ are different form one another when $R^4$ is p-methoxybenzyl and Y is methylene, are manufactured.

3. A process according to claim 1, characterized in that compounds in accordance with claim 1 in which $R^1$ is hydroxy, lower-alkanoyloxy, benzoyloxy, phosphonooxy, lower-alkoxycarbonyloxy; $R^2$ is lower-alkoxy or lower-alkenyloxy; $R^3$ is lower-alkoxy; $R^4$ is substituted phenyl; X is oxygen or sulphur and Y is methylene or imino are manufactured.

4. A process according to claim 3, characterized in that compounds of formula I in which Y is imino are manufactured.

5. A process according to claim 1, characterized in that 4-ethoxy-2-hydroxy-6-methoxy-N-(p-methoxybenzyl)-benzamide is manufactured.

6. A process according to claim 1, characterized in that 2-[(p-anisylamino)carbonyl]-5-ethoxy-3-methoxyphenyl dihydrogen phosphate and its disodium salt are manufactured.

7. A process according to claim 1, characterized in that 1-(4-ethoxy-2-hydroxy-6-methoxyphenyl)-3-(4-methoxyphenyl)-1-propanone is manufactured.

8. A process according to claim 1, characterized in that 5-ethoxy-3-methoxy-2-[3-(p-methoxyphenyl)-propionyl]-phenylacetate is manufactured.

9. A process according to claim 1, characterized in that 2-hydroxy-4,6-dimethoxy-N-[p-(methylthio)benzyl]-benzamide is manufactured.

10. A process according to claim 1, characterized in that 2-{[[p-(allyloxy)benzyl]amino]carbonyl}-3,5-dimethoxyphenyl benzoate is manufactured.

11. A process according to claim 1, characterized in that 1-(4-ethoxy-2-hydroxy-6-methoxyphenyl)-3-(4-methoxyphenyl)-1-propanethione is manufactured.

12. A process according to claim 1, characterized in that 5-ethoxy-3-methoxy-2-[3-(4-methoxyphenyl)-propionyl]-phenyl ethylcarbonate is manufactured.

13. A process according to claim 1, characterized in that 2-hydroxy-6-methoxy-4-propoxy-N-(p-methoxybenzyl)-benzamide is manufactured.

14. A process according to claim 1, characterized in that 4-(allyloxy)-2-hydroxy-6-methoxy-N-(p-methoxybenzyl)-benzamide is manufactured.

15. A process according to claim 1, characterized in that 2-hydroxy-6-methoxy-4-(3-methyl-2-butenyloxy)-N-(p-methoxybenzyl)benzamide is manufactured.

16. A process according to claim 1, characterized in that disodium 2-[(p-anisylamino)-carbonyl]-3-methoxy-5-propoxyphenyl phosphate is manufactured.

17. A process according to claim 1, characterized in that 4-ethoxy-2-hydroxy-6-methoxy-N-(p-methoxybenzyl)-benzamide is manufactured.

**Revendications pour les Etats contractants: BE, CH, DE, FR, GB, IT, LI, LU, NL, SE**

1. Composés de formule

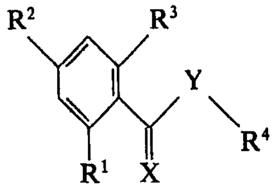

(I)

où X représente un oxygène, un soufre ou un hydroxyimino; Y représente un méthylène ou un imino; $R^1$ représente un hydroxy, un phosphonooxy, un glycosyloxy, un glycosyloxy acylé, un acyloxy ou un alcoxy inférieur-carbonyloxy, $R^2$ représente un alcoxy inférieur, un alcényloxy inférieur ou un alcoyle inférieur-thio; $R^3$ représente un alcoxy inférieur; $R^4$ représente un alcoyle inférieur, un p-alcoxy inférieur-benzoyle ou un phényle substitué ou non substitué, un benzyle, un pyridylméthyle, un furfuryle, un tétrahydrofurfuryle, un thiényle, un tétrahydrothiényle, un pyrrolylméthyle, un pyrrolinylméthyle ou un pyrrolidinyl-méthyle, avec cette condition que Y n'est pas un méthylène lorsque $R^4$ est un alcoyle, un phényle, un phényle substitué, un benzyle ou un p-hydroxybenzyle et avec la condition supplémentaire que $R^2$ et $R^3$ sont différents l'un de l'autre lorsque $R^4$ est un p-méthoxybenzyle et Y un méthylène,

et leurs sels pharmaceutiquement acceptables.

2. Composés selon la revendication 1, où X représente un oxygène, un soufre ou un hydroxyimino, Y représente un méthylène ou un imino; $R^1$ représente un hydroxy, un phosphonooxy, un glycosyloxy, un glycosyloxy acylé, un acyloxy ou un alcoxy inférieur-carbonyloxy, $R^2$ représente un alcoxy inférieur ou un alcoyle inférieur-thio, $R^3$ représente un alcoxy inférieur, $R^4$ représente un alcoyle inférieur, un p-alcoxy inférieur-benzoyle ou un phényle substitué ou non substitué, un benzyle, un pyridylméthyle, un furfuryle, un tétrahydrofurfuryle, un thiényle, un tétrahydrothiényle, un pyrrolylméthyle, un pyrrolinylméthyle, ou un pyrrolidinylméthyle avec cette condition que Y n'est pas un méthylène lorsque $R^4$ est un alcoyle, un phényle, un phényle substitué, un benzyle ou un p-hydroxybenzyle, et avec cette condition supplémentaire que $R^2$ et $R^3$ sont différents l'un de l'autre lorsque $R^4$ est un p-méthoxybenzyle et Y est un méthylène.

3. Composés selon la revendication 1, où $R^1$ est un hydroxy, un alcanoyloxy inférieur, un benzoyloxy, un phosphonooxy; un alcoxy inférieur-carbonyloxy, $R^2$est un alcoxy inférieur ou un alcényloxy inférieur, $R^3$ est un alcoxy inférieur, $R^4$ est un phényle substitué, X est un oxygène ou un soufre et Y est un méthylène ou un imino.

4. Composés selon la revendication 3, où Y est un imino.

5. 4-éthoxy-2-hydroxy-6-méthoxy-N-(p-méthoxybenzyl)-benzamide.

6. Phosphate diacide de 2-[(p-Anisylamino)carbonyl]-5-éthoxy-3-méthoxyphényle et son sel disodique.

7. 1-(4-éthoxy-2-hydroxy-6-méthoxyphényl)-3-(4-méthoxyphényl)-1-propane.

8. 5-ethoxy-3-méthoxy-2-[3-(p-méthoxyphényl)-propionyl]phényl-acétate.

9.2-hydroxy-4,6-diméthoxy-N[p-(méthylthio)benzyl]-benzamide.

10. 2-{[[p-(allyloxy)benzyl amino]carbonyl}-3,5-diméthoxyphényl-benzoate.

11. 1-(4-ethoxy-2-hydroxy-6-méthoxyphényl)-3-(4-méthoxyphényl)-1-propanethione.

12. 5-ethoxy-3-méthoxy-2-[3-(4-méthoxyphényl)propionyl]phényl-éthylcarbonate.

13. 2-hydroxy-6-méthoxy-4-propoxy-N(p-méthoxybenzyl)-benzamide.

14. 4-(allyloxy)-2-hydroxy-6-méthoxy-N-(p-méthoxybenzyl)benzamide.

15. 2-hydroxy-6-méthoxy-4-(3-méthyl-2-buténylοxy)-N-(p-méthoxybenzyl)benzamide.

16. Phosphate disodique de 2-[p-anisylamino)-carbonyl]-3-méthoxy-5-propoxyphényle.

17. 4-éthoxy-2-hydroxy-6-méthoxy-N-(p-méthoxybenzyl)-benzamide.

18. Composés de formule I comme agents contre les maldies virales.

19. Procédé de préparation de composés de formule I, caractérisé en ce que

a) on hydrogène un composé de formule

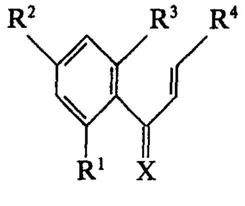

(II)

où X, $R^1$, $R^2$, $R^3$, et $R^4$ sont tels que dans la formule I,

en présence d'un catalyseur dans un solvant, ou

b) on fait réagir un dérivé réactif d'un acide carboxylique de formule

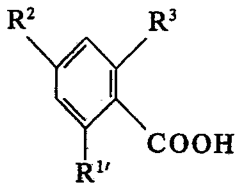

(III)

où $R^2$ et $R^3$ sont tels que dans la formule I et $R^{1'}$ est un groupe hydroxy protégé, avec un composé de formule

$R^4-NH_2$ (IV)

où $R^4$ est tel que dans la formule I, puis on sépare le groupe protecteur du groupe hydroxy, ou

c) on fait réagir une acétophénone de formule

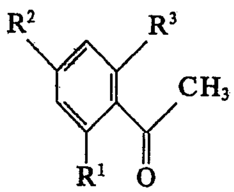

(V)

où $R^1$, $R^2$, et $R^3$ sont tels que dans la formule I, avec de l'iode dans une amine tertiaire et on fait réagir le sel obtenu avec une amine de formule IV ci-dessus ou

d) on transpose un ester de formule

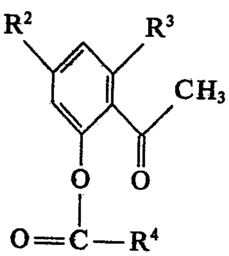

(VI)

où $R^2$, $R^3$ et $R^4$ sont tels que dans la formule I, en présence d'une base dans un solvant, ou

e) on fait réagir une cétone de formule

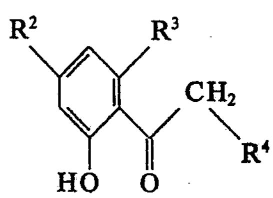

(VII)

où $R^2$, $R^3$ et $R^4$ sont tels que dans la formule I, avec un sel d'hydroxylamine dans un solvant, ou

f) on fait réagir un composé carbonyle de formule

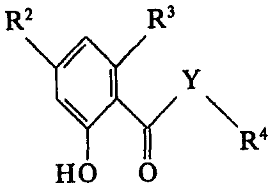

(VIII)

où Y, $R^2$, $R^3$ et $R^4$ sont tels que dans la formule I,
avec du pentasulfure de phosphore en présence d'une base dans un solvant, ou

g) on fait réagir un phénol de formule

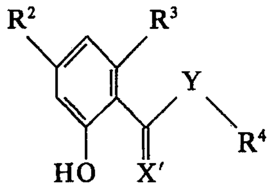

(IX)

où Y, $R^2$, $R^3$ et $R^4$ sont tels que dans la formule I et X' est un oxygène ou un soufre.
avec un agent acylant en présence d'une base, ou

h) on fait réagir un phénol de formule IX ci-dessus avec de l'oxychlorure de phosphore ou du dibenzylphosphorachloridate en présence d'une base dans un solvant et on hydrolyse ou selon les cas hydrogénolyse le produit de la réaction et si on le désire on transforme un composé obtenu en un sel, ou

i) on fait réagir un phénol de formule IX ci-dessus avec un halogénure de glycosyle ou un halogénure de glycosyle acylé en présence d'un catalyseur dans un solvant et si on le désire on retire ensuite les groupes acyle.

20. Préparations pharmaceutiques caractérisées en ce qu'elles contiennent un composé de formule I.

**Revendications pour l'Etat contractant: AT**

1. Procédé de préparation de composés de formule

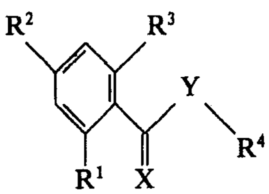

(I)

où X représente un oxygène, un soufre ou un hydroxyimino; Y représente un methylène ou un imino, $R^1$ représente un hydroxy, un phosphonooxy, un glycosyloxy, un glycosyloxy acylé, un axyloxy ou un alcoxy inférieur-carbonyloxy; $R^2$ représente un alcoxy inférieur, un alcényloxy inférieur ou un alcoyle inférieur-thio; $R^3$ représente un alcoxy inférieur; $R^4$ représente un alcoyle inférieur, un p-alcoxy inférieur-benzoyle ou un phényle substitué ou non substitué, un benzyle, un pyridylméthyle, un furfuryl, un tétrahydrofurfuryle, un thiényle, un tétrahydrothiényle, un pyrrolylméthyle, un pyrrolinylméthyle ou un pyrrolidinylméthyle, avec cette condition que Y n'est pas un méthylène lorsque $R^4$ est un alcoyle, un phényle, un phényle substitué, un benzyle ou un p-hydroxybenzyle et avec la condition supplémentaire que $R^2$ et $R^3$ sont différents l'un de l'autre lorsque $R^4$ est un p-methoxybenzyle et Y un méthylène,
et de leurs sels pharmaceutiquement acceptables,

a) on hydrogène un composé de formule

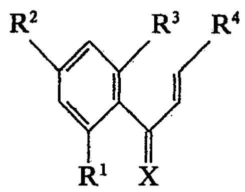

(II)

où X, $R^1$, $R^2$, $R^3$, et $R^4$ sont tels que dans la formule I, en présence d'un catalyseur dans un solvant, ou

b) on fait réagir un dérivé réactif d'un acide carboxylique de formule

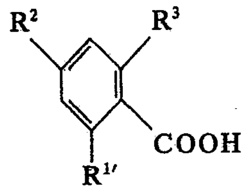

(III)

où $R^2$ et $R^3$ sont tels que dans la formule I et $R^{1'}$ est un groupe hydroxy protégé, avec un composé de formule

$R^4—NH_2$ (IV)

où $R^4$ est tel que dans la formule I, puis on sépare le groupe protecteur du groupe hydroxy, ou

c) on fait réagir un acétophénone de formule

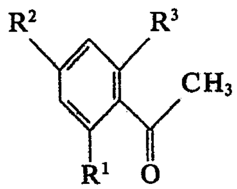

(V)

où $R^1$, $R^2$ et $R^3$ sont tels que dans la formule I, avec de l'iode dans une amine tertiaire et on fait réagir le sel obtenu avec une amine de formule IV ci-dessus, ou

d) on transpose un ester de formule

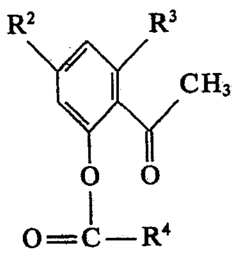

(VI)

où $R^2$, $R^3$ et $R^4$ sont tels que dans la formule I, en présence d'une base dans un solvant, ou

e) on fait réagir une cétone de formule

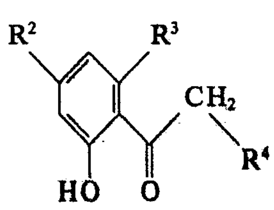

(VII)

où $R^2$, $R^3$ et $R^4$ sont tels que dans la formule I, avec un sel d'hydroxylamine dans un solvant, ou

f) on fait réagir un composé carbonyle de formule

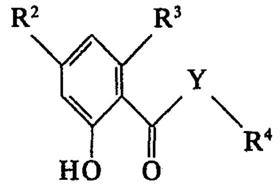

(VIII)

où Y, $R^2$, $R^3$ et $R^4$ sont tels que dans la formule I, avec du pentasulfure de phosphore en présence d'une base dans un solvant, ou

g) on fait réagir un phénol de formule

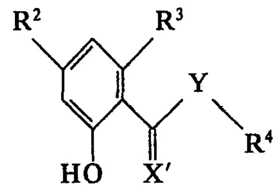

(IX)

où Y, $R^2$, $R^3$ et $R^4$ sont tels que dans la formule I et X' est un oxygène ou un soufre, avec un agent acylant en présence d'une base, ou

h) on fait réagir un phénol de formule IX ci-dessus avec de l'oxychlorure de phosphore ou du dibenzylphosphorachloridate en présence d'une base dans un solvant et on hydrolyse ou selon les cas hydrogénolyse le produit de la réaction et si on le désire on transforme un composé obtenu en un sel, ou

i) on fait réagir un phénol de formule IX ci-dessus avec un halogénure de glycosyle ou un halogénure de glycosyle acylé en présence d'un catalyseur dans un solvant et si on le désire on retire ensuite les groupes acyle.

2. Procédé selon la revendication 1, caractérisé en ce en ce qu'on prépare des composés selon la revendication 1, où X représente un oxygène, un soufre ou un hydroxyimino, Y représente un méthylène ou un umino, $R^1$ représente un hydroxy, un phosphonooxy, un glycosyloxy, un glycosyloxy acylé, un acyloxy ou un alcoxy inférieur-carbonyloxy, $R^2$ représente un alcoxy inférieur ou un alcoyle inférieur-thio; $R^3$ représente un alcoxy inférieur; $R^4$ représente un alcoyle inférieur, un p-alcoxy inférieur-benzoyle ou un phényle substitué ou non substitué, un benzyle, un pyridylméthyle, un furfuryle, un tétrahydrofurfuryle, un thiényle, un tétrahydrothiényle un pyrrolylméthyle, un pyrrolinylméthyle ou un pyrrolidinylméthyle; avec cette condition que Y n'est pas un méthylène lorsque $R^4$ est un alcoyle, un phényle, un phényle substitué, un benzyle ou un p-hydroxybenzyle, et avec cette condition supplémentaire que $R^2$ et $R^3$ sont différents l'un de l'autre lorsque $R^4$ est un p-méthoxybenzyle et Y est un méthylène.

3. Procédé selon la revendication 1, caractérisé en ce qu'on prépare des composés selon la revendication 1, où $R^1$ est un hydroxy, un alcanoyloxy inférieur, un benzoyloxy, un phosphonooxy, un alcoxy inférieur-carbonyloxy; $R^2$ est un alcoxy inférieur ou un alcényloxy inférieur; $R^3$ est un alcoxy inférieur; $R^4$ est un phényle substitué; X est un oxygène ou un soufre et Y est un méthylène ou un imino.

4. Procédé selon la revendication 3, caractérisé en ce qu'on prépare des composés de formule I où Y est un imino.

5. Procédé selon la revendication 1, caractérisé en ce qu'on prépare le 4-éthoxy-2-hydroxy-6-méthoxy-N-(p-méthoxybenzyl)-benzamide.

6. Le phosphate diacide de 2-[(p-anisylamino)carbonyl]-5-éthoxy-3-méthoxyphényle et son sel disodique.

7. La 1-(4-éthoxy-2-hydroxy-6-méthoxyphényl)-3-(4-méthoxyphényl)-1-propanone.

8. Le 5-éthoxy-3-méthoxy-2-[3-(p-méthoxyphényl)-propionyl]phényl-acétate.

9. Le 2-hydroxy-4,6-diméthoxy-N-[p-(méthylthio)benzyl]-benzamide.

10. Le 2-{[[p-(allyloxy)benzyl]amino]carbonyl}-3,5-diméthoxyphényle-benzoate.

11. La 1-(-4-éthoxy-2-hydroxy-6-méthoxyphényl)-3-(4-méthoxyphényl)-1-propanthione.

12. Le 5-éthoxy-3-méthoxy-2-[3-(4-méthoxyphényl)-propionyl]phényl-éthylcarbonate.

13. Le 2-hydroxy-6-méthoxy-4-propoxy-N-(p-méthoxybenzyl)-benzamide.

14. Le 4-(allyloxy)-2-hydroxy-6-méthoxy-N-(p-méthoxy-benzyl)benzamide.

15. 2-Hydroxy-6-méthoxy-4-(3-méthyl-2-buténoyloxy)-N-(p-méthoxybenzyl)benzamide.

16. Le phosphate disodique de 2-[p-anisylamino)-carbonyl]-3-méthoxy-5-propoxyphényle.

17. Procédé selon la revendication 1, caractérisé en ce qu'on prépare le 4-éthoxy-2-hydroxy-6-méthoxy-N-(p-méthoxybenzyl)-benzamide.